# EUROPEAN PATENT SPECIFICATION

(11) **EP 3 215 181 B1**
(45) Date of publication and mention of the grant of the patent: **18.12.2024**
(21) Application number: 15804327.3
(22) Date of filing: 05.11.2015
(51) Int. Cl.: A61K 38/48, A61P 1/18, A61K 47/44, A61K 47/34, A61K 9/20, A61K 9/14, A61K 38/54, A61P 1/00, A61P 3/10

(54) **PROCESSES FOR PRODUCING COMPOSITIONS WITH IMPROVED SAFETY PROFILE COMPRISING PANCREATIN AND COMPOSITIONS SUITABLE FOR PHARMACEUTICAL USE**
VERFAHREN ZUR HERSTELLUNG VON ZUSAMMENSETZUNGEN MIT PANKREATIN MIT VERBESSERTEM SICHERHEITSPROFIL UND ZUSAMMENSETZUNGEN, WELCHE ZUR PHARMAZEUTISCHEN VERWENDUNG GEEIGNET SIND
PROCÉDÉS POUR PRODUIRE DES COMPOSITIONS À PROFIL DE SÉCURITÉ D'EMPLOI AMÉLIORÉ COMPRENANT DE LA PANCRÉATINE ET DES COMPOSITIONS ADAPTÉES À UN USAGE PHARMACEUTIQUE

(30) Priority: 05.11.2014 EP 14191832; 01.10.2015 EP 15187823
(43) Date of publication of application: 13.09.2017
(73) Proprietor: Abbott Laboratories GmbH, 30173 Hannover (DE); Abbott GmbH & Co. KG, 65205 Wiesbaden (DE)
(72) Inventor: SHLIEOUT, George, 30173 Hannover (DE); BREITENBACH, Jörg, 30173 Hannover (DE); RUPP, Christopher, 30173 Hannover (DE); RÜFFER, Frauke-Regina, 30173 Hannover (DE); SCZESNY, Frithjof, 30173 Hannover (DE)
(74) Representative: Vossius & Partner Patentanwälte Rechtsanwälte mbB
(86) International application number: PCT/EP2015/075787
(87) International publication number: WO 2016/071434

(56) References cited:
- EP-A2- 0 131 740
- WO-A1-2005/092370
- WO-A1-2007/014896
- WO-A1-2008/101344
- WO-A1-2011/032907
- US-A- 5 410 022
- SCHULZE-MAKUCH ET AL: "Surfactant-modified zeolite can protect drinking water wells from viruses and bacteria", EOS, vol. 83, no. 18, 30 April 2002 (2002-04-30), pages 193 - 204, XP002739267, Retrieved from the Internet <URL:http://onlinelibrary.wiley.com/doi/10.1029/2002EO000128/epdf> [retrieved on 20150505]
- BREITENBACH J ED - LEHR CLAUS-MICHAEL DAUM NICOLE SCHNEIDER MARC SCHAFER ULRICH F: "Melt extrusion: from process to drug delivery technology", EUROPEAN JOURNAL OF PHARMACEUTICS AND BIOPHARMACEUTICS, ELSEVIER SCIENCE PUBLISHERS B.V., AMSTERDAM, NL, vol. 54, no. 2, 1 September 2002 (2002-09-01), pages 107 - 117, XP004377352, ISSN: 0939-6411, DOI: 10.1016/S0939-6411(02)00061-9
- DHENGE RANJIT M ET AL: "Twin screw wet granulation: Effects of properties of granulation liquid", POWDER TECHNOLOGY, vol. 229, 1 January 2012 (2012-01-01), pages 126 - 136, XP028931441, ISSN: 0032-5910, DOI: 10.1016/J.POWTEC.2012.06.019
- CASSIDY CORONA M ET AL: "Development of novel oral formulations prepared via hot melt extrusion for targeted delivery of photosensitizer to the colon.", PHOTOCHEMISTRY AND PHOTOBIOLOGY 2011 JUL-AUG, vol. 87, no. 4, July 2011 (2011-07-01), pages 867 - 876, XP002753671, ISSN: 1751-1097

## Description

The present application relates in a first aspect to processes or methods for producing compositions with lipase-activity which have an improved safety profile, comprising subjecting certain mixtures, comprising at least (a) a biological material derived from human or mammalian animal origin which has lipase activity, and (b) a surfactant-component, comprising at least one surfactant, to process variants selected from melt granulation, melt pelletization and melt extrusion, at defined temperatures and for defined time-periods. By virtue of the surfactants and the processing parameters as further detailed herein, the processes or methods substantially diminish the concentration of unwanted biological contaminants which may be present in the biological starting material, in particular certain viruses, while retaining the desired biological activity of said biological material, in particular its desired enzyme activity like its lipase activity. Said biological material derived from a human or mammalian animal source is an enzyme or enzyme-mixture with i.a. lipase activity, such as pancreatin and/or a pancreatin-containing mixture of digestive enzymes, in particular porcine pancreatin.

In a second aspect the present invention relates to solid or semi-solid compositions comprising
(a) an enzyme or an enzyme mixture having lipase activity, namely pancreatin and/or a pancreatin-containing mixture of digestive enzymes; (b) a surfactant-component comprising surfactant(s), co-surfactant(s) and preferably a lipophilic phase; (d) a polymeric additive and (c) further auxiliaries. Said compositions are suitable for phannaceutical use. Said solid or semi-solid compositions is prepared by the processes and methods disclosed herein.

Still further described herein are pharmaceutical compositions comprising the said compositions with lipase-activity. The pharmaceutical compositions as described herein may in particular be administered to humans, preferably by oral administration

### 1. Background

The risk of biological, in particular viral contamination is a feature common to products derived from human or animal, in particular mammalian, origin, such as pancreatin of porcine origin. While other biological contaminants such as bacteria or protozoa may be present in the starting material, these are usually inactivated during established manufacturing processes for products designated for human use. Still there is a need for better and more effective methods to inactivate more resistant biological contaminants like medium to highly resistant viruses and in particular highly resistant viruses such as non-enveloped viruses, in products for human use, be it only as a precautionary safety measure.

Known methods for viral inactivation include e.g. pasteurization, dry heat, vapour heat. solvent /detergent treatment and low pH. The selection of the methods to be employed for viral inactivation depend on the nature and contamination of the biological of interest of the product which is to be processed, the method of purification used and the nature of the viruses of concern. For example, solvent or detergent treatment can disrupt the lipid membrane of enveloped viruses and has thus been used for their inactivation. However, many non-enveloped viruses arc generally not inactivated by solvent or detergent treatment. Heat, in particular dry heat, is a known physical inactivation treatment for even highly resistant non-enveloped viruses in biological material whose desired biological properties shall be preserved, but only few such processes are known to be useful on an industrial scale where large quantities need to be processed in relatively short time periods at high efficiency and preserving desired biological activities. Known processes using dry heat on an industrial scale for reducing the concentration of more resistant viruses in products like enzymes for therapeutic use usually require extended time periods of several hours and monitoring of moisture content to avoid compromising the desired biological activity of the product of interest, e.g. the desired enzymatic activities present in porcine pancreatin (see e.g. WO 2007/014896 A1 and EP 2 255 086 A1).

In WO 2005/092370 A1 pharmaceutical compositions comprising enzyme mixtures with lipase activity are described which may be prepared by mixing the enzymes with surfactants, whereby the mixture may be processed by melt extrusion, melt granulation or melt pelletization at temperatures of about 50 °C. While the described processing conditions retain good lipolytic activity it has been found by virus-spiking experiments (not disclosed in WO 2005/092370) that the processing conditions described therein were insufficient for inactivating more resistant viruses like medium-resistant viruses, medium to highly resistant viruses or highly resistant viruses. In particular, no significant inactivation of viruses such as of the highly resistant *porcine Parvovirus* ("PPV") may be expected if contaminated compositions are subjected to conditions described in WO 2005/092370 A1.

EP 864 326 A2 relates to processing methods of i.a. pancreatin in matrices not comprising self-emulsifying systems.

Pancreatin (pancrelipase) in accordance with the EU (US) pharmacopoeias, is a pancreatic extract containing several digestive enzymes whose properties are defined by standard monographs such as in the European (*Ph. Eur*., see monograph 350 "pancreas powder") or US (*USP*) Pharmacopoeias. Pancreatin is derived from mammalian animal pancreas glands and comprises i.a. the excretory pancreatic enzymes lipase, alpha-amylase and the proteases trypsin and chymotrypsin, as well as other enzymes. Pancreatin for pharmaceutical use is typically of bovine or porcine origin, whereby porcine pancreatin is preferred. Pancreatin from porcine pancreases for therapeutic use is manufactured according to strictly controlled processes only from pigs declared suitable for human consumption under the supervision of veterinarians.

Pharmaceutical compositions comprising pancreatin (panerclipase) such as Creon^{®} are used to supplement digestive enzymes in the treatment and/or the prophylaxis of maldigestion in mammals, in particular humans, and in particular of maldigestion due to chronic exocrine pancreatic insufficiency such as in patients suffering from cystic fibrosis, chronic pancreatitis or patients who have undergone upper gastrointestinal surgery.

### 2. Summary

Provided herein are, in a first aspect, processes as claimed in appended claims 1 to 8 for the preparation of a solid or semi-solid composition, preferably for pharmaceutical use, and comprising a biological material derived from a human or mammalian animal origin, being pancreatin or pancreatin-containing mixtures of digestive enzymes derived from a mammal, and defined surfactant-components. Said processes are suitable to substantially reduce unwanted biological contaminants which may be or may have been present in the biological material before the process, e.g. to levels acceptable to health authorities for pharmaceutical products. Unwanted biological contaminants which can be reduced by the processes as described herein comprise in particular viruses, including enveloped and non-enveloped viruses with different degrees of resistance, e.g. medium resistant viruses, medium-highly resistant viruses and highly resistant viruses. At the same time, said processes are suitable to retain to the largest extent the desired activities of the biological material used, in particular the desired and therapeutically valuable enzymatic activities of pancreatin or pancreatin-containing mixtures of digestive enzymes (e.g. lipolytic, amylolytic and/or proteolytic activities). For example, the processes as described herein are suitable to produce pharmaceutical compositions comprising pancreatin or pancreatin-containing mixtures with improved in vivo lipolytic efficiency and acid-stabilization as e.g. described in WO 2005/092370 A1, with the additional benefit of a robust biological safety profile.

Moreover, due to their high efficiency, the processes as described herein are suitable for industrial use as they allow producing e.g. compositions of pancreatin or pancreatin-containing mixtures of digestive enzymes for pharmaceutical use on an industrial scale while only exposing said compositions to elevated temperatures for significantly shorter time periods than the processes and methods known from the prior art (see e.g. WO 2007/014896 A1 or EP 2 255 086 A1).

As the processes described herein usually do not involve the use of water or other solvents they are generally useful for processing any moisture-sensitive biological material derived from a tissue of human or mammalian animal origin, including e.g. porcine pancreatin.

In one embodiment, the present invention therefore provides a process for the preparation of a composition as claimed in appended claims 1 to 8.

Further provided herein are. in a second aspect, solid or semi-solid compositions comprising (a) a biological material derived from a human or mammalian animal origin like an enzyme or enzyme-mixture having lipase activity, in particular pancreatin or pancreatin-containing mixtures of digestive enzymes derived from a mammal, (b) defined surfactant-components, (c) one or more antioxidant(s) in a concentration of 50 to 200 ppm and (d) polymeric additives. Said compositions are optimized with regard to their processibility in the processes as described herein, with the effect of an improved biological safety profile while preserving high levels of enzymatic activities and showing excellent mechanical properties, even at elevated temperatures as occurring in warmer climate zones. Said solid or semi-solid compositions furthermore exhibit similar advantageous properties as the compositions disclosed in WO 2005/092370 A1 with regard to increased lipolytic efficiency and stabilization in the acidic pH range after administration to patients. In preferred alternatives, the solid or semi-solid compositions are pharmaceutical compositions, preferably pharmaceutical compositions for oral use.

In embodiments, the present invention therefore also provides solid or semi-solid compositions comprising:
(a) an enzyme or enzyme mixture having lipase activity, in particular pancreatin and/or a pancreatin-containing mixture of digestive enzymes deriv ed from a mammal;
(b) a surfactant-component of
   (i) at least one surfactant
   (ii) at least one co-surfactant and
   (iii) preferably a lipophilic phase; and
(c) one or more pharmaceutically acceptable auxiliary agent(s) and
(d) a polymeric additive selected from hydrophilic polymers with melting points or glass transition temperatures of 50-160 °C. more in particular of 50-70 °C or of 50-65 °C;
whereby the weight-to-weight ("w/w") ratio of additive (d) and surfactant-component (b) is 0 4 (2.5) to 1.5 (3.2). preferably 1 (1:1) to 1,33 (2:1.5), and most preferably 1 (1:1)
as claimed in appended claims 9 to 16.

Said solid or semi-solid compositions as herein described is produced by the processes as described herein and their preferred variants.

Additionally the present invention provides pharmaceutical compositions comprising a composition in accordance with the above, optionally comprising one or more pharmaceutically acceptable excipients and the use thereof as claimed in appended claims 17 and 18.

The following terms and abbreviations used herein shall have the meanings as explained here below:
"API" as used herein stands for "active pharmaceutical ingredient". The preferred API as disclosed herein is pancreatin, in particular porcine pancreatin as generally used for therapeutic purposes, i.e. pancreatin according to the requirements of standard pharmacopoeias, e.g. *Ph. Eur.* And/or *USP* and suitable for oral administration in the treatment or prophylaxis of maldigestion in mammals, in particular humans, and in particular of maldigestion due to chronic exocrine pancreatic insufficiency such as in patients suffering from cystic fihrosis, chronic pancreatitis or patients who have undergone upper gastrointestinal surgery. Porcine pancreatin for therapeutic use usually has a residual moisture content of not more than 5 % by weight, preferably of not more than 3.5 % by weight, in each case relative to the total weight of the porcine pancreatin, as measured by the method of the Ph. Eur. (loss on drying). Due to the nature of the process as described herein it is suited for e.g. porcine pancreatin of even lower moisture contents than 3.5 % by weight (w/w) (loss on drying).

As used herein, the term "biological material derived from animal origin" includes mammalian and non-mammalian animals (such as avian and insect), while mammalian animal origin, in particular porcine or bovine origin is preferred and porcine origin is most preferred.

As used herein, the term "comprises" or "comprising" is meant to comprise the meaning of "consisting of".

The terms "enzyme" and "enzyme-mixture" as used herein refer in particular to mammalian pancreatic enzymes, pancreatin or pancrelipase of mammalian anima) origin, in particular of bovine or porcine origin. Key enzymes for oral therapeutic use of mammalian pancreatic enzymes comprise lipase(s), protcase(s) and amylase(s), as is known in the art.

As used herein, the term "extrudate" refers to a composition which has been processed through and shaped by melt extrusion. Typically, the extrudate leaves an extruder at the die side. The extrudate has usually the same composition as the melt composition.

The term "melt composition" as used herein refers to the mixture comprising biological material (in particular pancreatin) (a), surfactant-component (b), optionally one or more auxiliary agent(s) (c) and optionally one or more polymeric additive(s) (d), softened by heat. The melt composition has usually the same composition as the extrudate.

The term "melted mass" as used herein refers to surfactant-component (b), optionally comprising one or more auxiliary agent(s), (c) and optionally comprising one or more polymeric additive(s). (d), softened by heat.

The term "minimal residence time" as used herein refers to the minimal amount of time the melt composition (including the API) is in an extruder (in particular a twin screw extruder) from the inlet port of the extruder for the API to the orifice (die), e.g. to determine the minimal time period which is needed to achieve robust inactivation of certain virus types while at the same time minimizing any losses in desired biological activity. As will be understood, the minimal residence time varies and can be adjusted in known manner as a function of e.g. the size of the extruder, applied screw speed, screw configuration and feed rate. In contrast to the mean residence time or the average residence time, the minimal residence time is determined as the time period when a tracer substance (e.g. curcumin or Sicovit^{®} red) which has been added to (and mixed with) the API first appears at the die site. This time period can be determined in known manner, e.g. visually, i.e. hy determining the time period between feeding the API with the marker substance to the extruder until the marker substance first appears at the die side of the extruder (i.e. measuring the time period which the API needs to be transported in the extruder from the inlet port to the die side). This period can also be determined by determining the onset of the curve for the known (calculated) residence time distribution, as is known in the an and described herein.

The terms "pancreatic enzymes", "pancreatin" and "pancrelipase" as used herein refer to enzymatic mixtures derived from mammalian pancreatic glands comprising digestive enzymes such as lipase, protease and amylase as main components. In particular, the terms "pancreatic enzymes", "pancreatin" and "pancrelipase" may be used synonymously herein and refer to pancreatic extracts suitable for therapeutic use, in accordance with standard pharmacopoeias, which contain several digestive enzymes whose properties are defined by standard monographs as explained above. Due to standard manufacturing processes, "pancreatic enzymes", "pancreatin" and "pancrelipase" are usually provided in powder form as "pancreatin powder", sometimes also referred to as "pancreas powder". Pancreatic enzymes, pancreatin and pancrelipase can also and preferably be APIs. Pancreatin is a natural product by its origin from animal, in particular mammalian sources. It is known that natural products can be subject to certain variabilities in their exact properties when used in e.g. industrial processes as the processes described herein. These variabilities may occur between different batches from the same supplier (depending on the sources from which the pancreatic glands come), or may occur between batches from different suppliers, e.g. due to certain differences in manufacturing processes. Although pancreatin products for therapeutic use are obtained in highly reproducible and standardized qualities, certain variabilities in processing properties between different pancreatin batches may nonetheless occur. Such variabilities may lead to e.g. variations in the processes and composition as described herein for optimal performance and processibility of e.g. about +/- 5 % by weight of pancreatin. Pancreatin suitable for the processes, methods and compositions as described herein can e.g. be obtained from Nordmark Arzneimittel GmbH & Co. KG, Uetersen, Germany; Scientific Protein Laboratories (SPL), Waunakee, Wisconsin, USA: Abbott Laboratories GmbH, Neustadt. Germany; or can be prepared according to known methods (see e.g. EP 115 023) or methods similar to these.

As used herein, the term "pharmaceutical composition" means a composition comprising the product obtained by the process as described herein and optionally one or more pharmaceutically acceptable excipients.

As used herein the term "pharmaceutically acceptable" refers to those compounds, materials, compositions and/or dosage forms, which are, in the scope of sound medicinal judgment, suitable for contact with the tissue of mammals, especially humans, without excessive toxicity. irritation, allergic response and other problem complications commensurate with a reasonable beneficial ratio.

As used herein the term "is processed at a temperature" means that the melt composition itself is processed at said temperature as a result of total energy applied. For melt granulation methods, the total energy applied is usually the heat energy provided, e.g. by an oil bath. For melt extrusion methods, the total energy applied is usually a combination of (i) heat energy as provided by the barrel of the extruder and (ii) mechanical energy, as a function of e.g. screw speed, throughput rate, resulting torque and shear forces. Usually, the melt composition (extrudate) can therefore be assumed to have the temperature at which the mixture for the preparation of a solid composition is to be processed. Suitable temperatures or temperature ranges as provided herein for melt extrusion process variants are usually measured as temperatures of the product (extrudate, melt composition) at the exit (die side) of the extruder, typically directly behind the die where the extrudate leaves the extruder. Temperatures are usually measured with an infrared thermometer as described in section "product temperature".

The terms "product temperature" and "extrudate temperature" are used herein to mean, for melt extrusion process variants, the temperature of the homogenized and extruded material in die barrel of a homogenizing extruder, measured at the die exit, by using a suitable thermometer, e.g. a calibrated infrared thermometer (Testo 845). Typically, the temperature of one extrudate or extrudate strand is measured several times (e.g. 3 to 5 times) where the highest temperature displayed is used in each case. The mean value of said highest temperatures from the several measurements is then recorded as the relevant temperature. Compositions of product and extrudate correspond to the composition of the mixture for the preparation of a solid composition, as described herein. For melt granulation processes and melt pelletization processes, the product temperature and/or the temperature of the melt composition can be directly measured with an inserting thermometer, c.g a digital inserting thermometer (Testo 720).

The term "solid (oral) composition" "solid composition" or "solid or semi-solid composition" as used herein is intended to encompass compositions suitable for (per-) oral administration having a defined outer shape, i.e. is also intended to encompass soft and/or semi-solid compositions, including e.g. lozenges.

As used herein, the time period applied for processing the mixture for the preparation of a solid or semi-solid composition refers to the time period during which said mixture is to be exposed to the prescribed processing temperatures (product or extrudate temperatures) in order to achieve the desired effects as described herein (both maximized reduction of biological contaminants and maximized retained desired biological activity of the biological material). For discontinuous process variants (e.g. discontinuous melt granulation, discontinuous melt pelletization), the suitable time period for processing said mixture is usually the time period measured as holding time at a desired product temperature once this has been reached. For semi-continuous (e.g. batch-wise melt extrusion) or continuous (e.g. continuous melt extrusion) process variants, the suitable time period for processing said mixture is preferably the time period measured as minimal residence time as described herein.

As used herein, numerical indications provided in the format of ranges or intervals, e.g of temperatures as "50-160 °C" or of weight percent as "2-90 wt.-%" are meant to designate (with the exception of explicitly measured values, e.g. as provided in the examples section) any value (integers, fractions) included within the given range or interval, including the range limits, the latter comprising the limits of usual rounding rules. E.g. a temperature range of "50-160 °C" will include i.a. any of 49.5 °C. 50 °C, 52.2 °C, 55.5°C and 160.4 °C. The term "% by weight" may be abbreviated to "wt.-%" herein.

The energy for arriving at the defined process temperatures of any embodiment of the process as disclosed herein can be applied as thermal energy (heating) or as mechanic energy (e.g. stirring, kneading) or by a combination of thermal energy and mechanic energy. In preferred embodiments, the mixture for preparing a solid composition is homogenized before processing and/or during processing. Homogenization can be done by any suitable method, e.g. by shaking, stirring or kneading. Homogenization by using a homogenizing extruder is preferred. Homogenization methods can at the same time serve to supply energy for arriving at the defined process temperatures. Preferred embodiments of the process disclosed herein use melt granulation, melt pelletization or melt extrusion technologies. Melt extrusion technologies are more preferred.

As provided herein, the lower limits of processing time and processing temperatures are selected so that viral loads of medium resistant viruses, medium-highly resistant viruses and in preferred embodiments even highly resistant viruses can significantly be reduced in the biological material derived from a tissue of human or animal origin, preferably in the pancreatin and/or a pancreatin-containing mixture of digestive enzymes, of the final composition when compared with the viral load of that same composition (product) before the process. Any upper limits of processing time and processing temperatures provided herein are selected so that desired key biological activities are preserved at acceptable levels in the biological material derived from a tissue of human or animal origin of the final composition when compared with the key biological activities of that same product before the process. Where the biological material derived from a tissue of human or animal origin is an enzyme or enzyme-mixture with at least lipase activity, e.g. pancreatin, key biological activities are lipolytic activity, amylolytic activity and/or proteolytic activity. Determination of the minimal and maximum processing times (residence times) to achieve an appropriate reduction of viral load on the one hand and on the other hand maintaining the enzymatic activity of key enzymes within the desired ranges and thus appropriately adjusting the process parameters is within the ability of the person of skill in the art based on its general knowledge and the information provided in the present specification.

### 3. Detailed description

In a first aspect, the present invention thus provides a process for the preparation of a solid or semi-solid composition, preferably for pharmaceutical use, comprising processing a mixture, comprising
(a) 40-75 % by weight of pancreatin and/or a pancreatin containing mixture of digestive enzymes derived from a mammal:
(b) 10-50 % by weight of a surfactant-component comprising
   (i) 2-90 % by weight, relative to the surfactant-component, at least one surfactant,
   (ii) 5-60 % by weight, relative to the surfactant-component, at least to one co-surfactant, and
   (iii) 0-70 % by weight, relative to the surfactant component, a lipophilic phase,
(c) one or more antioxidant(s) present in a concentration of 50-200 ppm relative to the total weight of the solid or semi-solid composition, and
(d) 0-35 % by weight of a polymeric additive selected from hvdrophilic polymers with melting points or glass transition temperatures of 50 - 70°C.

and wherein the % by weight of components (a), (b), (c) and (d) are w/w of the mixture for preparing a solid composition and add to 100 % by weight for said mixture in each case;
by a method selected from melt granulation, melt pelletization and melt extrusion, in each case at a product temperature of 90-130 °C and for a time period of not less than 30 sec. and not exceeding 45 min as claimed in appended claim 1.

Preferred variants of the processes for the preparation of the solid or semi-solid composition comprise the following process steps:
aa) Preparing the mixture for processing by mixing components (a), (b), (c) and (d) in amounts and ratios as required to arrive at a desired target composition. Mixing of the components can be done in different ways, e.g. as described herein in more detail for process variants melt granulation, melt pelletization and melt extrusion.
bb) Introducing energy into the mixture. By introducing energy into the mixture, the temperature of the mixture is increased. Temperature increase usually results in plastification of the mixture Introducing energy into the mixture can be done in different ways, e.g. by heating and/or application of mechanical energy, as described herein in more detail for process variants melt granulation, melt pelletization and melt extrusion.
cc) Shaping the plastified mixture. Shaping the plastified mixture, e.g. the plastified mixture as obtained in process step bb), can be done in different ways, e.g. as described herein in more detail for process variants melt granulation, melt pelletization and melt extrusion. The plastified mixture may be shaped into forms which are suitable for producing pharmaceutical dosage forms, e.g. extrudate strands, or may be directly shaped into pharmaceutical dosage forms, e.g. pellets, granules or powders. Forms which are suitable for producing pharmaceutical dosage forms can be further processed into pharmaceutical dosage forms, e.g. by breaking extrudate strands into pellets (actively or passively) and/or or by rounding rough pellets into spheres, pellets or micropellets of spherical or nearly spherical shape.
dd) Reducing introduction of energy into the mixture By reducing introduction of energy into the mixture (including stopping introduction of energy), the mixture will usually solidify while maintaining its shape, e.g. its shape as resulting from process step cc). Reducing introduction of energy into the mixture may therefore result in solid or semi-solid compositions as described herein.
ee) Optionally collecting the solid or semi-solid compositions obtained from the process and/or optionally further processing the solid or semi-solid compositions to pharmaceutical dosage forms as described herein in more detail.

In preferred variants of the processes, process steps aa) - ee) are earned out in the order given above.

In preferred process variants, the mixture for preparing a solid or semi-solid composition is homogenized before processing and/or during processing.

In further preferred process variants, the compositions or extrudates formed in the process or as a result of the process are subsequently processed into granules, granulates, pellets, spheres, tablets and/or powders.

Component (a) is preferably present in an amount of 40-75 wt.-% of said mixture, more preferably in an amount of 40-70 wt.-%, 45-68 wt.-%, or 47-68 wt.-%. In other preferred embodiments, component (a) is present in an amount of 50-70 wt.-% of said mixture, more preferred in an amount of 58-70 wt.-% (64 wt.-% +/- 6 wt.-% ) and yet more preferred in an amount of 60-68 wt.-%.

Surfactant-component (b) is preferably present in an amount of 10-50 wt.-% of said mixture, preferably in an amount of 15-45 wt.-%, more preferably in an amount of 15-30 wt.-% or in an amount of 15-25 wt.-% .

Component (d), the polymeric additive, can be present in an amount of 0-35 wt.-% of said mixture, preferably in an amount of 5-35 wt.-%, more preferably in an amount of 10-30 wt.-% and even more preferred in an amount of 10-25 wt.-%.

The w/w-ratio between the polymeric additive component (d) and the surfactant-component (b) is between 0.4 (2:5) and 1.5 (3:2), preferably between 0.75 and 1.3. More preferably, their w/w-ratio is 1:1.

In one preferred embodiment of the processes as described herein, component (a) is porcine pancreatin in an amount of 64 % +/- 6 % by weight of the mixture and components (b), (d) and further optional auxiliary agents (c) together are present in an amount of 36 % +/- 6 % by weight of the mixture.

In other preferred embodiments of the processes as described herein, components (b) and (d) make up 30-42 wt.-% (36 wt.-% +/- 6 wt.-% ) of said mixture or composition and are composed of (b) semisynthetic lauroyl macrogol-32 glycerides on the basis of hydrogenated palm kernel oil having a melting point of about 42.5-47.5 °C (e.g. Gelucire^{®} 44/14) and (d) polyethylene glycol 4000, in a ratio of 1:1 w/w, and further comprise (c) 100-150 ppm, preferably 150 ppm butylatcd hydroxyanisolc (a known mixture of 2-*tert*-butyl-4-hydroxy-anisole and 3-*tert*-butyl-4-hydroxyanisole, abbreviated to "BHA" hereinafter), relative to the combined total weight of components (b) and (d).

The solid or semi-solid composition and/or the mixture for preparing a solid or semi-solid composition as described herein preferably comprise less than 1 wt.-% solvent (including water), relative to the total weight of the solid composition or mixture, and can be solvent-free.

Component (a), the pancreatin and/or a pancreatin containing mixture of digestive enzymes derived from a mammal, is preferably porcine pancreatin as generally used for therapeutic purposes, as explained herein in more detail.

The surfactant-component (b) comprises (i) at least one surfactant. A surfactant
is a chemical compound comprising at least two moieties, the first being hydrophilic and/or polar or ionic and having a high affinity to water and the second containing an aliphatic chain of greater or lesser length and being hydrophobic (lipophilic); i.e., a surfactant is usually amphiphilic. Surfactants having lower HLB ("hydrophilic-lipophilic balanec)-values are more hydrophobic (lipophilic), whereas surfactants having a higher HLB value are more hydrophilic (lipophobic). Surfactants suitable for use with the processes as described herein have an HLB value (according to the definition and method of Griffin) above 6 and below 18, preferably above 8 and below 16. Surfactants can be any surfactant suitable for use in a pharmaceutical composition and may be anionic, cationic, zwitterionic or non-ionic. Surfactants can be classified by virtue of their chemical structure. In general, chemical classes polyethylene glycol-fatty acid monoesters; polyethylene glycol-fatty acid diesters; polyethylene glycol glycerol fatty acid esters: ethylene glycol alkyl ethers: polyethylene glycol glycerol fatty acid esters: polyethylene glycol alkyl ethers; oligoethylene glycol alkyl ethers: polyethylene glycol sterol ethers: polyethylene glycol sorbitan fatty acid esters; sugar esters, in particular mono-, di- and/or tri-esters of sucrose with food fatty acids, e.g. suitable qualities of sucrose stearate, sucrose palmitate, sucrose laurate and/or sucrose oleate; d-α-tocophcryl polyethylene glycol 1000 succinate ("vitamin E TPGS"); and amphothoric compounds such as fatty acid amidoalkylbetaines with C₂-C₂₂ fatty acids, and their mixtures are suitable. Oligoethylcne glycols and their derivatives as described herein are meant to have a degree of polymerization (or an average degree of polymerization, as applicable) of the ethylene glycol moieties of 2-8 and comprise in particular di(ethylene glycol), tri(ethylene glycol), tetra(ethylene glycol), penta(ethylene glycol) and hexa(cthylene glycol). A representative but not limiting disclosure of preferred surfactants for use in the processes as described herein can be found on page 7, line 13 to page 10, line 31 of WO 2005/092370 A1, expressly including the disclosure on p. 15, lines 4-32.

Surfactants which can be used in the processes disclosed herein can be selected from (1) non-ionic surfactants, comprising polyethylene glycol fatty acid mono- and/or di-esters with aliphatic C₆-C₂₂ carboxylic acids; polyethylene glycol glycerol fatty acid esters with aliphatic C₆-C₂₂ carboxylic acids; polyethylene glycol alkyl mono- and/or di-ethers with aliphatic C₁₂-C₁₈ alcohols. oligoeihylene glycol ethers with aliphatic C₂-C₁₈ alcohols; and mixtures of any of the foregoing, and (II) ionic surfactants, comprising lecithin, lysolecilliin, phosphatidylcholine, phosphatidylethanolamine, phosphatidylglycerol, phosphatidylserine, lysophosphatidylcholine, lysophosphatidylethanolamine. lysophosphatidylglycerol. lyso-phosphatidylinositol. lysophosphatidic acid, lysophosphatidylscrine: and mixtures of any of the foregoing, and mixtures of any of the foregoing surfactants from (I) and (II).

Non-ionic surfactants are preferred. Of the ionic surfactants, lecithin is preferred.

Aliphatic carboxylic acids as described herein may also be referred to as "fatty acids" and can comprise saturated, unsaturated and polyunsaturated carboxylic acids which may have chain lengths of 4 to 22 carbon atoms, preferably of 6-22 carbon atoms.

Aliphatic alcohols as described herein can comprise saturated, unsaturated and polyunsaturated alcohols (where applicable) which may have chain lengths of 2 to 22 carbon atoms, e.g. 2-18 carbon atoms. 12-18 carbon atoms or 12-22 carbon atoms.

The surfactant-component (b) further comprises (ii) at least one co-surfactant. A co-surfactant or co-emulsificr as referred to herein is a chemical compound which has both hydrophobic (lipophilic) and hydrophilic moieties, but with the hydrophobic (lipophilic) nature predominating. It is intended to make the aqueous and oily phases in a micro-emulsion mutually soluble. Co-surfactants suitable for use with the process as described herein have a HLB value below 10, preferably below 8. and even more preferred below 6. Co-surfactants can be partial esters of of polyhydric/polyvalent alcohols like glycerol, propylene glycol and/or polyglycerols (such as diglycerol, triglycerol, tetraglycerol) with aliphatic carboxylic acids ("fatty acids"); esters of ethyl diglycol with aliphatic carboxylic acids: partial ethers of glycerol, propylene glycol, and/or polyglycerols with aliphatic alcohols ("fatty alcohols"); ethers of ethyl diglycol with aliphatic alcohols and mixtures of any of the foregoing. Co-surfactants can be classified by virtue of their chemical structure. In general, chemical classes such as mono-glycerides, polyglycerized fatty acids and propylene glycol fatty acid esters are suitable. A representative but not limiting disclosure of preferred co-surfactants for use in the process as described herein can be found on page 10. line 33 to page 12, line 6 of WO 2005/092370 A1, expressly including the disclosure on p. 15, lines 4-32.

In accordance with the present invention
co-surfactants can be selected from mono-acylglycerides with aliphatic C₆-C₂₂ carboxylic acids, mono-ethers of glycerol with aliphatic C₁₂-C₂₂ alcohols, partial esters of propylenglycol with aliphatic C₆-C₂₂ carboxylic acids, partial esters of polyglycerol with aliphatic C₆-C₂₂ carboxylic acids, oligoethylene glycol monoesters with aliphatic C₁₅-C₂₂ carboxylic acids, oligoethylene glycol diesters with aliphatic C₆-C₂₂ carboxylic acids and mixtures of any of the foregoing.

In preferred embodiments, the surfactant-component (b) further comprises (iii) a lipophilic phase. A lipophilic phase (also referred to as a lipidic phase) as referred to herein is a water immiscible substance, e.g. a water-immiscible liquid. As used herein, the lipophilic phase is preferably a diglyccride, a triglyceride and/or a mixture of a triglyceride and a diglyccride. Suitable lipohilic phases are preferably di- and triglycer ides of aliphatic carboxylic acids (fatty acids) with 4 to 22 carbon atoms, in particular 6 to 22 carbon atoms, and mixtures thereof. A representative but not limiting disclosure of preferred lipophilic phases for use in the process as described herein can be found on page 12, line 8 to page 13, line 24 of WO 2005/092370 A1. expressly including the disclosure on p. 15, lines 4-32.

More preferred lipophilic phases can be selected from di- and triacylglycerides with aliphatic C₆-C₂₂ carboxylic acids or mixtures thereof.

Several commercially available surfactant and/or co-surfactant compositions can contain small to moderate amounts of di- and triglycerides, typically as a result of an incomplete reaction of a triglyceride starting material in, e.g., a transesterification reaction. Examples of such compositions are e.g. disclosed on page 13, line 26 to page 15, line 32 of WO 2005/092370 A1 and may be suitable to provide in addition to the surfactant (i) and /or co-surfactant (ii) part of the mixture (b) all or part of the lipophilic component/phase (iii).

Preferred commercially available compositions comprising surfactant, co-surfactant and/or lipophilic phases comprise e.g. propylenglycol caprylates (CAS RN: 85883-73-4, 85883-73-4). available e.g. as Capryol^{®}, and glyceryl monolitioleate (CAS RN: 68424-61-3), available c.g. as Maisine^{®}, all from Gattefossé, Lyon, France.

In more preferred variants of surfactant-component (b) for all embodiments of processes and compositions as described herein may be a mixture comprising the following components:
(i) at least one surfactant, selected from the group consisting of: polyethylene glycol monoesters with aliphatic C₆-C₂₂ carboxylic acids: polyethylene glycol diesters with aliphatic C₆-C₂₂ carboxylic acids; polyethylene glycol glycerol esters with aliphatic C₆-C₂₂ carboxylic acids; polyethylene glycol alkyl monucthers with aliphatic C₁₂-C₁₈ alcohols; polyethylene glycol alkyl diethers with aliphatic C₁₂-C₁₈ alcohols, oligoethylene glycol ethers with aliphatic C₂-C₁₈ alcohols; lecithin: lysolecithin; phosphatidylcholine, phosphatidylethanolamine: phosphatidylglycerol; phosphatidyl-serine; lysophosphatidylcholine; lysophosphatidylethanolamine; lysophosphatidyl-glycerol: lysophosphatidylinositol; lysophosphatidic acid; lysophosphatidylserinc or mixtures of any of the foregoing;
   preferably in an amount of 2 to 90% wt.-% of the surfactant-component,
(ii) at least one co-surfactant, selected from the group consisting of: mono-acylglycerides with aliphatic C₆-C₂₂ carboxylic acids; mono-ethers of glycerol with aliphatic C₁₂-C₂₂ alcohols: partial esters of propylenglycol with aliphatic C₆-C₂₂ carboxylic acids; partial esters of polyglycerol with aliphatic C₆-C₂₂ carboxylic acids, oligoethylene glycol monoesters with aliphatic C₆-C₂₂ carboxylic acids, oligocthylene glycol diesters with aliphatic C₆-C₂₂ carboxylic acids or mixtures of any of the foregoing;
   preferably in an amount of 5 to 60 wt.-% of the surfactant-component and
(iii) a lipophilic phase selected from the group consisting of: diacylglycerides with aliphatic C₆-C₂₂ carboxylic acids, triacylglycerides with aliphatic C₆-C₂₂ carboxylic acids or mixtures of any of the foregoing;
   preferably in an amount of 0 to 70% wt.-% of the surfactant-component.

Preferred variants of surfactant components (b) for all embodiments of processes and compositions as described herein may comprise 2-90 wt.-%, more in particular 40-90 wt.-% and even more in particular 60-85 wt.-% surfactants (i); 5-60 wt.-%, more in particular 5-40 wt.-% and even more in particular 15-30 wt.-% co-surfactants (ii); and 0-70 wt.-%, more in particular 5-40 wt.-%, even more in particular 15-30 wt.-% lipophilic phase (iii), all wt.-% relative to the total weight of surfactant-component (b) and adding to 100 % in each case.

Preferred as surfactant-component (b) are mixtures of surfactant and/or co-surfactant which can - e.g. due to incomplete (transesterification) reactions of the starting material used for their manufacture - also comprise amounts of di- and triglycerides (i.e., a lipophilic phase as used herein) and therefore can represent complete systems composed of surfactant, co-surfactant and lipophilic phase. Such complete systems which can represent surfactant-component (b) are e.g. known as Self Microcmulsifying Drug Delivery Systems (SMEDDS^{®}) and are e.g disclosed in WO 95/08983 (equivalent to US 6312704) or WO 99/44589 (equivalent to US 2003/021844). Preferred surfactant-components (b) of this type are oleoyl macrogol-6 glycerides EP (CAS RN: 97488-91-0, 9004-96-0, 69071-70-1, 68424-61-3), e.g. commercially available as Labrafil^{®} M1944CS; lauroyl polyoxyl-6 glycerides (CAS RN: 93334-20-4, 9004-81-3, 57107-95-6, 27638-00-2), e.g. commercially available as Labrafil^{®} M2130 CS; lineoyl macrogol-6 glycerides EP (CAS RN: 85536-08-9, 9004-96-0, 85536-08-9, 61789-25-1), commercially available as Labrafil^{®} M2125CS; or caprylocaproyl polyoxylglycerides (CAS RN: 85536-07-8, 84963-88-2, 223129-75-7, 85409-09-2, 73398-61-5, 61791-29-5), commercially available as Labrasol^{®}, all from Gatterossé, Lyon, France.

Particularly preferred complete systems which can represent surfactant-component (b) are semisynthetic lauroyl macrogol-32 glycerides on the basis of hydrogenated palm kernel oil having a melting point of about 42.5-47.5 °C and comprising mono- and diesters of poly ethylene glycol ("PEG"s) 1500 at about 72 wt.-%, mono-, di- and triglycerides of fatty acids 20 wt.-% and free PEG 1500 about 8 wt.-%. (apportionment of the fatty acids: C8< 15 wt.-%. C10< 12 wt.-%, C12<30-50 wt.-%, C14 5-25 wt.-%, C16 4-25 wt.-% C8 5-35 wt.-%), free glycerol < wt.-3% commercially available as Gelucire^{®} 44/14 (CAS RN: 93334-20-4, 9004-81-3); and semisynthetic stearoyl macrogol-32 glycerides having a melting point of about 46-51 °C and comprising mono- and diesters of PEG 1500 at about 72 wt.-%, mono-, di- and triglycerides of PEG 1500 20 wt.-%, and free PEG 1500 at about 8 wt.-% (apportionment of the fatty acids: C8<3 wt.-%, C10<3 wt.-%, C12< 5 wt.-%. C14<5 wt.-%, C16 40-50 wt.-%, C18 48-58 wt.-%), free glycerol <3 wt.-%, commercially available as Gelucire^{®} 50/13 (CAS RN: 91744-66-0. 9004-99-3), all from Gattefossé, Lyon, France. Gelucire^{®} 44)14 is most preferred.

The mixture for the preparation of a solid or semi-solid composition further comprises (c) one or more antioxidant(s).

Carriers include, without limitation, polyols such as mannitol, sorbitol, xylitol: disaccharides such as lactose, sucrose, dextrose and maltose: polysaccharides such as maltodcxtrine and dextranes: starches such as corn starch; celluloses such as microcrystallinc cellulose, sodium carboxy methylccllulose, low-substituted hydroxypropyl cellulose, hydroxyl ethyl cellulose, hydroxypropyl cellulose or mixtures thereof; cylodextrines and inorganic agents such as dicalcium phosphate, calcium hydrogen phosphate; hydroxyapatite, tricalcium phosphate, talcum and silica. Microcrystalline cellulose, sucrose and/or lactose are preferred as carriers.

Suitable antioxidants include, without limitation, ascorbic acid, a-tocopherol, resveratrol, carotinoides, propylgallate, octylgallate, dodecylgallatc, tert.-butylhydrochinone, BHA and butylhydroxytoluenc. BHA is preferred as antioxidant.

Suitable binding agents include, without limitation, hydroxypropylmethyl cellulose (HPMC). hydroxypropyl cellulose (HPC), prcgelatinzed starch and combinations thereof, preferably HPMC.

Disintegrants, include, without limitation, carboxymethylccllulosc calcium (CMC-Ca). carboxymethylcellulose sodium (CMC-Na), crosslinked PVP (e.g., crospovidone, Polyplasdone^{®} or Kollidon^{®} XL), alginic acid, sodium alginate, guar gum, cross-linked CMC (croscarmellose sodium, e.g. Ac-Di-Sol^{®}), carboxymethyl starch-Na (sodium starch glycolate) (e.g., Primojel^{®} or Explotab^{®}), preferably crosslinked PVP and/or croscarmellose sodium.

Lubricants include, without limitation, magnesium stearate, aluminium or calcium silicate, stearic acid, hydrogenated castor oil, talc, glyceryl behenate, sodium stearate fumarate and combinations thereof, preferably magnesium stearate.

Glidants include, without limitation, colloidal SiO₂ (e.g., Aerosil^{®} 200), magnesium trisilicate, powdered cellulose, talc and combinations thereof, preferably colloidal SiO₂

Component (c) as used herein are antioxidant(s) preferably BHA. Antioxidants, in particular BHA, are present in a concentration of 50 to 200
ppm, more preferably of 100 to 150 ppm, in particular of 1 50 ppm, relative to the total weight of the melted mass.

In preferred embodiments of the processes as described herein, the mixture for the preparation of a solid or semi-solid composition comprises a polymeric additive (d). Mixtures of one or more polymeric additives are comprised. Preferably, the polymeric additive is selected from hydrophilic polymers with melting points or glass transition temperatures of 50-160 °C. more preferably of 50-70 °C, or of 50-65 °C.

Suitable hydrophilic polymers may preferably be selected from polyoxyalkylenes, poloxamers. polyvinyl pyrrolidones ("PVP"s) and/or polyvinylpyrrolidone-vinyl acetate copolymers.

In preferred embodiments, said polymeric additive is a hydrophilic polymer selected from polyethylene glycols and poloxamers or a mixture of said hydrophilic polymers. Preferably PEGs and/or poloxamers which have an average molecular weight of 3000-30000 g/mol, more preferably of 3250-25000 g/mol, can be used, such as PEG 4000, PEG 8000, PEG 20000 and/or poloxamer 188 (the latter being commercially available as Kolliphor^{®} 188 from BASF SE, Ludwigshafen. Germany), Also preferred are hydrophilic polymers which have an average molecular weight of 3250-15000 g/mol and more preferable of 3500-9000 g/mol. In a most preferred embodiment, the polymeric additive is PEG 4000. PEGs of different average molecular weights are commercially available from a variety of suppliers, e.g. from Merck Chemicals GmbH, Darmstadt. Germany.

Suitable hydrophilic polymers can also be polyvinyl pyrrolidone ("PVP"), in particular PVP grades with a nominal K-value (measured as 1 %- or 5 %- w/volume solutions in water) of not less than 16, e.g. Kollidon^{®} 17PF, Kollidon^{®}, 30 or Kollidon^{®} 90 F; and/or polyvinylpyrrolidone-vinyl acetate copolymers, in particular copovidone, e.g. Kollidon^{®} VA64, all of the foregoing commercially available from BASF SE.

In a preferred embodiment of the processes as described herein, the mixture for preparing a solid or semi-solid composition comprises:
(a) 40-75 wt.-%, preferably 40-70 wt.-% of the pancreatin and/or pancreatin containing mixture of digestive enzymes derived from a mammal;
(b) 10-50 wt.-%, preferably 15-45 wt.-% of the surfactant-component,
(c) 50 to 200 ppm of one or more antioxidant(s), and
(d) 5-35 wt.-%, preferably 10-30 wt.-% of the polymeric additive,
wherein the wt.-% of components (a), (b), (c) and (d) are w/w of the mixture for preparing a solid arc semi-solid composition and add to 100 % by weight for said mixture in each case.

In a more preferred embodiment of the process for the preparation of a solid composition as described herein, the mixture for preparing a solid composition is particularly suited for melt-extrusion or twin-screw melt granulation and comprises:
(a) 50-75 wt.-%, preferably 50-70 wt.-% of porcine pancreatin,
(b) 15-30 wt.-% of the surfactant-component comprising:
   (i) 2-90 wt.-%, relative to the surfactant-component, of the at least one surfactant,
   (ii) 5-60 wt.-%, relative to the surfactant-component, of the at least one co-surfactant and
   (iii) 0-70 wt.-%, relative to the surfactant-component, of the lipophilic phase, wherein the percentages (i). (ii) and (iii) add to 100 wt.-% for the surfactant-component in each case,
(c) 50 to 200 ppm of one or more antioxidant(s), and
(d) 10-25 wt.-% of the at least one hydrophilic polymer which has a melting point or glass transition temperature of 50-160 °C,
wherein the wt.-% of components (a). (b), (c) and (d) are w/w of the mixture for preparing a solid composition and add to 100 wt.-% for said mixture in each case.

In another more preferred embodiment, the mixture for preparing a solid composition) is particularly suited for melt-extrusion, twin-screw melt extrusion or twin-screw melt granulation and comprises:
(a) 50-75 wt.-%, preferably 50-70 wt.-% of porcine pancreatin;
(b) 15-30 wt.-% of a surfactant-component comprising:
   (i) 2-90 wt.-% , relative to the surfactant-component, of at least one surfactant selected from polyethylene glycol monoesters with aliphatic C₆-C₂₂ carboxylic acids, polyethylene glycol diesters with aliphatic C₆-C₂₂ carboxylic acids; polyethylene glycol glycerol esters with aliphatic C₆-C₂₂ carboxylic acids: polyethylene glycol alkyl monoethers with aliphatic C₁₂-C₁₈ alcohols, polyethylene glycol alkyl diethers with aliphatic C₁₂₋C₁₈ alcohols, oligoethylene glycol ethers with aliphatic C₂-C₁₈-alcohols; lecithin and mixtures of any of the foregoing,
   (ii) 5-60 wt.-% , relative to the surfactant-component, of at least one co-surfactant, selected from mono-acylglycerides with aliphatic C₆-C₂₂ carboxylic acids, mono-ethers of glycerol with aliphatic C₁₂-C₂₂ alcohols, partial esters of propylenglycol with aliphatic C₆-C₂₂ carboxylic acids and/or partial esters of polyglycerol with aliphatic C₆-C₂₂ carboxylic acids, oligoethylene glycol monoesters with aliphatic C₆-C₂₂-carboxylic acids and/or, oligoethylene glycol diesters with aliphatic C₆-C₂₁-carboxylic acids and mixtures of any of the foregoing, and
   (iii) 0-70 wt.-% , relative to the surfactant-component, of a lipophilic phase, selected from diacylglycerides with aliphatic C₆-C₂₂ carboxylic acids; triacylglycerides with aliphatic C₆-C₂₂ carboxylic acids and mixtures of any of the foregoing, wherein the percentages (i), (ii) and (iii) add to 100 wt.-% for the surfactant-component in each case,
(c) 50 to 200 ppm of one or more antioxidant(s),
(d) 10-25 % at least one polymeric additive, which is a hydrophilic polymer having a melting point or glass transition temperature of 50-110°C,
wherein the wt.-% of components (a), (b), (c) and (d) arc w/w of the mixture for preparing a solid composition and add to 100 wt.-% for said mixture in each case.

The mixtures for the preparation of a solid composition as described herein, in particular the preferred mixtures as defined herein, are usually thermoplastic and can be shaped into suitable forms at elevated temperatures. Said mixtures can contain components which have higher melting points or glass transition temperatures than the processing temperatures actually applied but can nonetheless be smoothly processed at the prescribed temperatures in melt granulation, melt pelletization and/or melt extrusion process variants. For example, mixtures as described herein may contain as polymeric additive(s) (c) certain PVP grades which have a glass transition temperature of higher than 130 °C, Such mixtures may nonetheless be processed at temperatures of below the glass transition temperatures of said PVP polymeric additives, e.g. at temperatures of 90-130 °C, as long as the mixture in its entirety is sufficiently softened or molten to be processed as described herein.

In the processes as described herein, the mixtures for preparing a solid or semi-solid composition are processed by a method selected from melt granulation, melt pelletization and melt extrusion. Melt granulation and melt extrusion arc preferred. Melt extrusion is most preferred.

In certain variants of the processes as described herein, the mixtures for preparing a solid or semi-solid composition are processed by melt pelletization to produce multi-particulate forms like granules, granulates, pellets, spheres and/or powders. In said melt pelletization processes, one or more meltable component(s) of the mixture for the preparation of the solid or semi-solid compositions, e.g. components (b), like Gelucire^{®} 44/14, and/or (c), like polyethylene glycol 4000, is/are mixed with the pancreatin and/or the pancreatin containing mixture of digestive enzymes derived from a mammal and the resulting mixture is then processed above the melting point (or glass transition temperature) of said meltable components) (in their entirety) by suitable process variants, e.g. centrifugal motion and/or heating. The processed mixture is then allowed to cool whereby the multi-particulate solid or semi-solid compositions are formed. Multi-particulate forms resulting from this process variant may further be processed to other oral administration forms like tablets, in known manner. Melt pelletization methods for direct pelletization processes are generally known, e.g. from WO 02/40045.

In preferred variants of the processes as described herein, the mixtures for preparing a solid or semi-solid composition are processed by melt granulation. The term "melt granulation" is used herein to describe a process whereby multi-particulate forms like granules granules, granulates, pellets, spheres and/or powders are obtained through the addition of either a motion binder or binding agent or a solid binder or binding agent which melts during the process. This process is also known as "melt agglomeration" or "thermoplastic granulation". It can be used to formulate active pharmaceutical ingredients for producing pharmaceutical compositions, usually for oral use (see e.g. Halle P.D. et al., Journal of Pharmacy and Phytothcrapeutics 1(3) (2013) 6-10). Multi-particulate forms resulting from this process variant may further be processed to other oral administration forms like tablets, in known manner.

In the melt-granulation process variants as described herein, the mixture for preparing a solid or semi-solid composition is preferably processed at a temperature (product temperature) of not less than 90 °C and not exceeding 125 °C, for a time period of not less than 180 sec. ( 3 min.) and not exceeding 30 min. (1800 sec.). In a more preferred variant of the melt-granulation process the mixture for preparing a solid or semi-solid composition is processed at a temperature of not less than 90°C and not exceeding 125 °C, for a time period of not less than 300 sec. ( 5 min.) and not exceeding 30 min. (1800 sec.). As explained herein in more detail, the term "is processed at a temperature" means that the mixture for the preparation of a solid or semi-solid composition (melt composition) itself is processed at said temperature as a result of the total energy applied.

In a general protocol for the melt granulation process variants as described herein, the components of the mixture for preparation of a solid or semi-solid composition as defined above are mixed in a suitable reaction vessel. e.g. a beaker, in desired quantities and ratios. Meltable components, e.g. components (b), like Gelucire^{®} 44/14, and/or (c), like polyethylene glycol 4000, may first be molten or softened at elevated temperatures. e.g. at temperatures of 40-60 °C, more in particular of 45-55 °C, e g. at 50 °C, Suitable
anti-oxidants, e.g. BHA, are added if desired and the resulting pre-mixture or melted mass may be homogenized in known manner, e.g. by stirring, for a time period of 1-15 min., preferably for 3-10 min., e.g. for 5 min. Stirring may be performed by any suitable means, e.g. spattles, known laboratory mixers or known intensive mixers. Component (a), e.g. porcine pancreatin in the form of pancreas powder for therapeutic use, may then be added at elevated temperatures, e.g. at the elevated temperatures described here before, e.g. at about 50 °C, and the resulting mixture may then be further homogenized, e.g. by stirring. Under continued stirring, the mixture may then be heated, e.g. by using an oil bath, to the target temperature (product temperature) of the process variant as described above, like to 90-130 °C, preferably to 90-125 °C, e.g. to 90 °C, The target temperature can be held for a desired period of time like 30-1800 sec., e.g. for 300 sec.

In embodiments of the melt-granulation processes, the said mixture is processed at a temperature of not less than 90°C not exceeding 130 °C for a time period of not less than 300 sec. and not exceeding 45 min.

In particularly preferred variants of the processes as described herein, the mixtures for preparing a solid or semi-solid composition are processed by melt extrusion. The term "melt extrusion" or "melt extruded" ("ME") is used herein to describe a process whereby a blended composition is heated and/or compressed to a molten or softened state and subsequently forced through an orifice, where the extruded product (extrudate) is formed into a shape in which it can solidify upon cooling. The blended composition is conveyed through one or more heating zones, usually by a screw mechanism. The screw or screws can be rotated by a variable speed motor inside a barrel, usually of cylindrical shape, where only a small gap may exist between the outside diameter of the screw and the inside diameter of the barrel. Due to the nature of the mixture used in the process described herein for the preparation of a solid composition, process variants using a twin screw extruder may also he referred to as "twin-screw granulation" or "twin-screw melt granulation". Melt extrusion technology and its application in the pharmaceutical industry is known per se, e.g. from Breitenbach. European Journal of Pharmaceutics and Biopharmaceutics 54 (2002) 107-117.

The melt extrusion processes as disclosed herein can expediently be carried out using an extruder, preferably a homogenizing extruder, like a single-screw extruder, a twin-screw-extruder, a triple:screw extruder or a planetary extruder. A twin-screw extruder, in particular a co-rotating twin-screw extruder, is preferred.

In melt extrusion process , said mixtures for preparing a solid or semi-solid composition are processed at a product temperature of of not less than 95 °C and not exceeding 125 °C (95-125 °C), more preferred at a product temperature of 90-120 °C, even more preferred at a product temperature of 100-110°C. In preferred alternatives the product (extrudate) temperature at the die side does not exceed 108 +/- 5 °C while processing.

In preferred alternatives of the described melt extrusion process variants said mixtures for preparing a solid or semi-solid composition are processed at the product temperature for a time period of not less than 30 sec. and not exceeding 30 min, preferably of not less than 40 sec. and not exceeding 20 min., more preferably of not less than 50 sec and not exceeding 10min and even more preferably of not less than 60 sec. and not exceeding 5 min (60-300 sec.). In further preferred embodiments said mixture is processed at the product temperature for a time period of 60-100 sec., more preferably of 80 +/- 15 sec., even more preferably of 83 +/- 5 sec. and still more preferably of 83 +/- 3 sec., all of the foregoing at the preferred temperatures as described in the previous paragraph.

In particularly preferred alternatives of die described melt extrusion process variants, said mixtures for preparing a solid or semi-solid composition are processed at a product temperature of not less than 90 °C and not exceeding 130 °C (90-130 °C) for a time period of not less than 30 sec. and not exceeding 30 min. In a preferred alternative, the said mixtures are processed at a product temperature of not less than 95 °C and not exceeding 125 °C (95-125 °C) for a time period of not less than 50 sec. and not exceeding 10 min. In another particularly preferred alternative, the said mixtures are processed at a product temperature of not less than 1 00 °C and not exceeding 120 °C (1 00-120 °C), in particular of 105-115 °C, for a time period of not less than 60 sec. and not exceeding 5 min

Suitable time periods for melt extrusion process variants (e.g. the specific embodiments, alternatives and variants as described here below) can preferably be measured as "minimal residence times". as herein described.

In one embodiment of the melt extrusion process variants as described herein, component (a) is first mixed with one or more meltable components (surfactant-component (b) and/or component (d)) to form a pre-mixture, then the pre-mixture so obtained is heated to produce a melt and finally the molten pre-mixlure is placed in the extruder (the "pre-mix feed" variant) for processing. In the extruder, the pre-mixture may be mixed with further components of the composition or may be processed without further mixing it with additional components. The pre-mix feed variant is used to run the process discontinuously (hatch-wise).

In another embodiment of the melt extrusion process variants as described herein, component (a) is directly fed into the extruder as a powder, powder mixture, compacted or granulated powder or powder mixture and is mixed in the extruder with a mixture of the surfactant-component (b) and the optional polymer additive (component (d)), wherein said mixture is in a molten or sufficiently softened state (the "direct feed" variant). Said mixture in a molten or sufficiently softened state may also comprise anti-oxidants in concentrutions sufficient to preserve the mixture against oxidation. The direct feed variant is usually preferred as it allows continuous processing.

Examples of preferred melt extrusion equipment suitable for melt extrusion process variants as described herein are provided here below:
In preferred embodiments, the melt extrusion equipment can typically be a known co-rotating twin screw extruder, containing a mixing/conveying zone, a heating/melting zone, and a pumping zone in succession up to the orifice (die). In the mixing/conveying zone, the powder blends are mixed and aggregates are reduced to primary particles by the shear force between the screw elements and the barrel. In the heating/melting zone, the temperature is at or above the melting point, glass transition temperature or softening temperature/softening temperature range of the melted mass or melt composition to sufficiently melt or soften it for smooth extrusion. In embodiments, the process as described herein can be a twin-screw melt granulation process, where a powdery API is mixed with the other components as described by the action of the twin-screw to produce a melted mass and this is then extruded through a sieve-like die plate.

Once in a sufficiently molten or softened state, the homogenized blend (melted mass) can then be pumped onto the screw bars through an inlet port after the powder feed. At the orifice (die). the melted mass can be formed into strands, cylinders or films. The extrudate that exits is then solidified, typically by a cooling process. Once solidified, the extrudate may then be further processed to form pellets, granules spheres, fine powder, and tablets.

Typical pilot plant extruders have diameters ranging from 18-30 mm, whereas extruders for production on industrial scale are typically larger. e.g. with diameters of not less than 50 mm. In the pharmaceutical field, melt extrusion equipment often comprises an extruder, downstream auxiliary equipment for extrusion and other monitoring tools used for performance and product quality evaluation.

Individual components within the extruder which are known per se and can typically be used with preferred process embodiments as described herein arc e.g.:
- Feed hopper (s) used to feed the material into the feed zone of the barrel;
- A temperature controlled barrel housing the screws of the extruder. Barrel sections can e.g. he heated by electric heaters or liquid. Barrel-cooling facilitates a temperature set point to maintain desired product (melt composition) temperature within the process section. Extruder barrels arc typically cooled by liquid and sometimes air. The most effective heat transfer design uses axial cooling bores inside the barrel and close to the process melt stream:
- Rotating screws: extruders used in the process as described herein preferably comprise two co-rotating screws inside a stationary cylindrical barrel ("co-rotating twin-screw extruder"). The extrusion screw is characterized by the "length-to-diameter ratio" or "L/D", expressing the length of the screw divided by the diameter, e.g. for extruders which have a modular design to facilitate variable screw configurations. Length of the screws in the extrusion process is often given in terms of L/D ratio (length of screw divided by screw diameter). Preferred screws to be used in processes as described herein have an L/D of at least 15:1, e.g. 25:1 or 32:1. Conventional screws known in the art can generally be used. Suitable screw lengths and screw configurations suitable for the process as described herein can readily be adapted by measures known in the art and additional disclosure as prov ided herein. In one preferred embodiment, a general set up (per screw) comprises at least one kneading block. preferably 2-8 kneading blocks. more preferably 4-6 kneading blocks , in each case over 4-6 L/D, whereby co-rotating screws arc preferred;
- Screw-driving unit(s);
- Dic(s): in processes as described herein, dies used in the process/extruder set-up can preferably be piercing dies having a hole diameter of 0.5 to 2.0 mm, preferably of 0.7 to 1.5 mm and more preferably of 0.8 mm

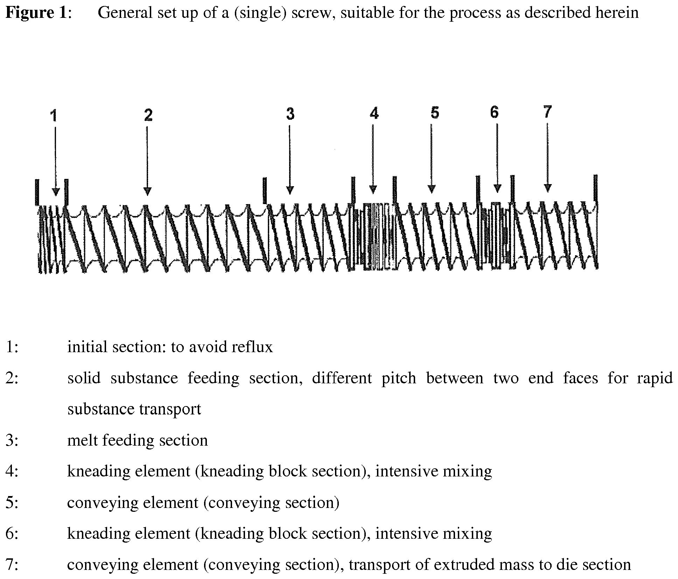

The melt extrusion process variants as described herein can preferably be carried out in a co-rotating twin screw-extruder, preferably additionally having kneading elements on the twin screws. Thus, the set up for a typical extrusion process may comprise the following elements known per se:
- One or more feed hoppers for gravimetric or volumetric feeding of powder or melted&liquid components: in preferred embodiments of the process as described herein, the API is fed as a powder, while the components of mixture b) are preferably fed by way of a liquid feeder;
- Temperature controlled barrels housing the screw bars:
- A conveying and kneading system (preferred) or conveying and kneading elements (preferred), respectively, for material transport and mixing. Screws to modify the residence time and to optimize the homogenization of the melted mass and/or the melt composition can be assembled from modules. A general set up showing the particular modules/sections of the screws (initial section to avoid reflux/solid feeding/melt liquid feeding/kneading clcment/conveying element) is shown in **Figure 1****.** Optimized combinations of such screw bar elements can e.g. he identified by performing DoE (Design of Experiments) studies on the basis of statistical programs as is known in the an, e.g. using software like "Design Expert^{®}" by Stat-Ease Inc., in its latest version.
- A die system for forming extrudates (preferably a sieve-like die), as well as
- Downstream auxiliary equipment (e.g., for cooling. pelletizing, spheronization, collecting), also in-line for continuous processing.

Preferred embodiments of the melt extrusion process variants as described herein comprise the following features:
- Endings of the conveying element (e.g. screw bar endings) at the die side preferably offer a flat (i.e. about 90°) and not a cubic or rounded end:
- The die does preferably not exhibit a die-channel, where the melt composition has to pass a certain distance without being actively forced or transported. A flat die design, such as a sieve-like die plate is therefore preferred. The resulting gap between the die site ending and the die site (die plate) should be minimized (< 1 mm) in order to minimize or avoid any dead volume at the die site which may lead to "de-mixing" of the mixture or segregation of its components. In preferred embodiments, the distance between the endings of the conveying element(s) (e.g. screw bar onding(s)) and the die side (e.g. sieve-like die plate or perforated disc) should not be more than 1 millimeter ("mm"), preferably not more than 0.5 mm, more preferably not more than 0.4 mm and still more preferred not more than 0.3 mm.

On a miniature-scale or laboratory-scale, e.g. a Three Tec twin screw-extruder 9 mm (ZE9) may be used in conjunction with the balanced feeder (loss in weight feeder) Three Tcc (ZD5) 5 mm powder feeder, a HNP pump (a feeder for liquids) and infrared (1R) temperature measuring equipment (e.g. Testo 845) which offers fast temperature measurement wherein min. and max. temperature values are updated at 100 ms time intervals.

For pilot scale processes, e.g. a Gabler DE-40 twin-screw extruder (diameter 40 mm), equipped for higher processing temperatures as needed, may be used in conjunction with the balanced feeder (loss in weight feeder) K-Tron, a HNP pump 024 (a feeder for liquids) and IR measuring equipment (temperature measurement) Testo 845.

For industrial scale processes, e.g. a Gabler DE- 100 (diameter 100 mm) extruder or a Gabler DE-120 (diameter 120 mm) extruder may be suitable, each equipped for higher processing temperatures as needed, optionally in combination with a Gabler Spheronizer R-400 or R-600.

Equipment as described above being available from Three Tee GmbH, Soon CH; Gabler GmbH & Co KG, Ettlingen DE, HNP Microsystemc GmbH, Schwerin DE; Coperion K-Tron Sewell, USA and Testo AG, Lenzkirch, DE.

Preferred melt extrusion process variables suitable for processes as described herein are described here below:
The "throughput rate" of the extruder characterizes the mass of the melt composition which passes the extruder in a given time interval. As will be understood, the throughput rate will be i.a. depending on the size/diameter of the extruder. In embodiments of the process as described herein, suitable throughput rates are e.g. about 2-3 g/min for a 9 mm extruder and about 133 g/min +/- 5% for a 40 mm extruder. At larger scales, in particular industrial scale the throughput rate may be e.g. 8 to 60 kg/h (133g/min +/- 5% up to 3600 g/min +/-5%) for a 100 mm extruder.

The "screw speed" is used to control the residence time. It will be understood that a suitable screw speed will need to be adapted to the scale of the extrusion process. In a preferred embodiment, in particular when using a Gabler D40 extruder, the screw speed is adjusted to 50 to 120 rpm, preferably to 75 +/- 15 rpm and most preferably to 70-80 rpm.

The term "residence time" as used herein refers to the time the melt composition is in an extruder (in particular a twin screw extruder) from the inlet port of the extruder for the API to the orifice (die). The residence time i.a. depends on the specific properties of the processed material (such as composition, flowability and viscosity) and may be determined upon equilibration of the process or process set up in accordance with methods known in the art such as by the methods described in Altomare et al.. Biotechnology Progress, 2 (3) (1986) 157-163, Gao et al., Polymer Engineering and Science, 40(1) (2000) 227-237, Poulesquen et al., Polymer Engineering and Science 43 (2) (2003) 1849-1862 and/or Dhenge et al., Powder Technology 229 (2012) 126-136. or references cited in any of the foregoing. In the literature it is common to indicate "average residence times" or "mean residence times". These can he calculated front an experimentally determined "residence time distribution" in known manner (see e.g. Dhengc et al.). For the process as described herein, the tracer used for determining the residence time may be a dye such as curcumin or Sudan red. Residence times (be it "minimal residence time", "average residence time" or "mean residence time") usually do not need to be determined or monitored permanently during production runs. Instead, once a residence time has been determined for a particular extruder, screw configuration, melt composition and parameter setup, it is usually sufficient to run the process without further monitoring and/or addition of a tracer. Should rc-calibration of the process be necessary, minimal residence time under any new or changed configuration can easily he determined again by the methods explained above.

Where the biological material derived from a tissue of human or mammalian animal origin is an enzyme or enzyme mixture with lipase activity, in particular pancreatin or a pancreatin-containing mixture derived from a mammal, tolerable residence times are a function of applied temperature and acceptable remaining enzyme (in particular lipase and amylase) activity after the extrusion process. Loss of key enzyme activity after the extrusion process when compared with the enzyme activity before the extrusion process of not more than about 30%, preferably not more than 25%, 20%, 15% or 10% should be aimed at. At industrial scale, the residence time should be such that the loss of key enzyme activity after the extrusion process does preferably not exceed 10-15%. In more preferred embodiments loss of key enzyme activity does not exceed 10 %.

As will be understood, the product temperature measured at the die side is a function of the temperature of the extruder's barrel and of the mechanical energy provided by the screws of the extruder. To arrive at and maintain a desired product temperature (measured as temperature of the extrudate leaving the extruder at the die side, usually by an IR thermometer), application of heat energy by heating of the barrel needs to be balanced with application of mechanical energy by the screws and screw setting/configuration applied. In the process as described herein, a desired temperature of the product (melt composition and/or extrudate) is usually achieved when the barrel temperature is typically kept in the range of 60-130 °C. In preferred embodiments, the extrusion is carried out at a barrel temperature of 80-130 °C. preferably at a barrel temperature of 80-120 °C, such as to ensure that the product temperature at the die is in the range of 90-130 °C or preferred temperature values as provided herein (see above). Adjusting desired product temperatures by coordinating relevant parameters, e.g. barrel temperature, screw configuration and/or screw speed is known in the art.

In preferred alternatives of the described melt extrusion process variants, the following parameters are applied or adjusted at a screw extruder, preferably a co-rotating twin-screw extruder:
- The feeding of the API onto the screw bar is performed by conveying elements offering a pitch of 1.5 L/D over a distance of 2.25 L/D;
- The melted mass is dosed into the extruder by conveying elements offering a pitch of 1 UD over a distance of 3 L/D after the API dosing port:
- The distance between API and melted mass dosing ports is 3.2 L/D;
- The first kneading zone is characterized by one kneading clement with a length of 1 L/D that offers live ridges, whereby each ridge is arranged in an angle of 45° to the following ridge. A reversed kneading element also offering an offset of 45° is placed on the screw bar after a further conveying segment;
- A conveying zone with a pitch of 0.75 and then 0.5 L/D is plugged on to the screw bar between the two kneading zones;
- in front of the die site the screw bar setup offers a conveying zone with two different pitches of 0.75 and then 0.5 L/D over a length of 3.25 L/D;
- The effective extrusion length offers a total length of 15 L/D or longer;
- Dₒ/Dᵢ ratio is 1.8;
- Dₒ/axis centre distance is 0.8:
- Dᵢ/axis centre distance is 1.5;
- The position of the feeding port for the API feeding is at 0-2.25 L/D;
- The position of the feeding port for the feeding of the melted mass is at 4-6 UD:
- The design of the sieve-like die plate has a ratio of blank surface (total surface of the die holes) to the total surface of the product contacting area 0.19 (number of holes within the die plate is 32 in the mini scale and 387 in pilot scale; die hole diameter is 1.0 mm for both scales; thickness of die plate is 1.0 mm).

"Dₒ" specifies the outer diameter of the screw/screw element of a (twin-) screw extruder. "D," specifies the inner diameter of the screw/screw element of a (twin-) screw extruder. The "Dₒ/Dᵢ ratio" refers to the outer-to-inner screw element diameter ratio of a (twin-) screw extruder. At low Dₒ/Dᵢ ratio very little free volume is available in the extruder, at higher Dₒ/Dᵢ ratios a higher extruder free volume is available allowing more material to be processed.

An exemplary screw bar set up indicating the different zones of the screw bar is shown in **Figure 1****.**

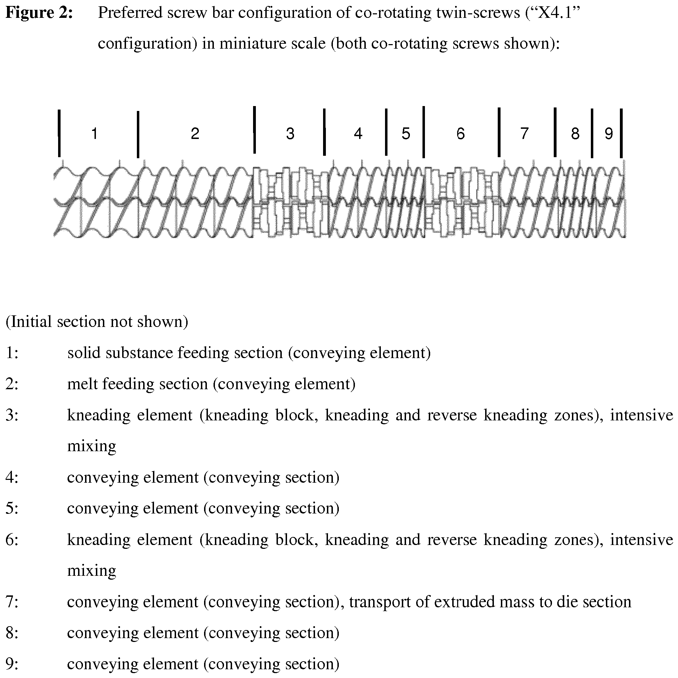

A preferred screw bar configuration is named "X4.1", Details of this configuration are shown in table 1 below. Further, a sketch of screw bar configuration X4.1 is shown in **Figure 2****.**

The compositions or extrudates formed by the processes as described herein may subsequently be further processed to granules, granulates, pellets, spheres, capsules, tablets or powders by methods known in the art. Extrudates may be broken up into small pieces (actively or passively) which can subsequently be rounded in a conventional rounding apparatus or spheronizer to provide pellets, granules or spheres of the desired size. Preferably, extrudates can be transferred into a conventional rounding apparatus and rounded to spheres of a diameter of 0.5-2 mm and regular shape as e.g. described in WO 2007/020259 A2 and WO 2007/020260 A2. Spheres of said desired dimensions and shape are also sometimes referred to as "microspheres" or "mini-microspheres".

Preferred embodiments of the processes as described herein comprise spheronizing the extrudate or extrudate fragments into pellets or spheres. Regulaily shaped pellets or spheres of suitable size, preferably with diameters of 0.5-2 mm, can then be obtained e.g. by spheronization in known manner. The extrudates obtained by the processes as described herein do usually not need to be cut into smaller fragments before spheronization as they would usually break up into smaller fragments, e.g. of about cylindrical shape, upon extrusion from the die. Before spheronizing, the extrudates may be cooled down, e.g. by aging them at room temperature or using a conveyor belt to cool down the extrudate by ambient air or by using active cooling before collecting them. Active cooling can be done by using a proper band equipped with a cooling mechanism which can be air and/or cooling with water pipes. The rounding or spheronization of the extrudates or extrudate fragments proceeds preferably after preheating a spheronizer at temperatures between, e.g., 35 and 56 °C. like at temperatures between 45 and 50 °C. The spheronized extrudate fragments may then be cooled by aging them at room temperature or using a conveyor belt for cooling by ambient air or by using active cooling, before collecting. In a particularly preferred embodiment, rope-like extrudates can be transformed into regular-shaped spheres (pellets) in a subsequent spheronization step, depending on the batch size. e.g.. in double jacket spheronizers such as Gabler Spheronizer R-250, Gabler Spheronizer R-400 or Gabler Spheronizer R-600 (all by Gabler, Ettlingen, Germany). Pellets or spheres whose diameters or any of their dimensions do not exceed 5 mm in length are often referred to as "minimicrospheres" or "micropellets". Minimicrospheres or micropellcts comprising porcine pancreatin and manufactured by the processes as described herein arc particularly preferred pharmaceutical forms.

In preferred embodiments, the rounded extrudates obtained after spheronization can be classified, e.g. by using known Kressncr sieves with approx. 0.7 mm screen size and approx. 1.6 mm screen size, with classifying yields of >/= 75%. ">/=" as used herein means "greater than or equal to".

In other embodiments, the processes as described herein may comprise a step wherein the obtained compositions or extrudates may be subsequently processed to other oral dosage forms, e.g. granules, tablets, coated tablets or powders.

In further embodiments, so obtained granules, pellets, spheres, tablets, coated tablets or powders can further be coated as desired with functional coatings, e.g. enteric coatings, or non-functional coatings, e.g. aesthetic coatings. In preferred embodiments, the so obtained granules, pellets, spheres, tablets, coated tablets or powders are not coated with enteric coatings.

**Table 1: Preferred screw bar set up (referred also as "X4. t" in the following)**

| **Screw bar** | **Type** | **Element length** | | **Pitch** | | **No. of elements** | **Total length (block)** | |
|---|---|---|---|---|---|---|---|---|
| **Element / section** | | **L/D** | | **L/D** | | | **L/D** | |
| *Distance cover and blocking element* | | | | | | | | *1.25* |
| Conveying (feeding API) | CE | | 0.75 | | 1.5 | 3 | | 2.25 |
| Conveying (feeding melted mass) | CE | | 1 | 4 | 1 | 3 | | 3 |
| Kneading block | KB | | 0.2 | | n.a. (45°) | 5 | | 1 |
| Reverse. kneading block | Rev. KB | | 0.2 | | n.a. (-45°) | 5 | | 1 |
| Conveying | Ch | | 0.75 | | 0.75 | 2 | | 1.5 |
| Conveying | CE | | 1 | | 0.5 | 1 | | 1 |
| Kneading block | KB | | 0.2 | | n.a. (45°) | 5 | | 1 |
| Reverse kneading block | Rev. KB | | 0.2 | . | n.a. (-45°) | 5 | | 1 |
| Conveying | CE | | 1.5 | | 0.75 | 1 | | 1.5 |
| Conveying | CE | | 1 | | 0.5 | 1 | | 1 |
| Conveying | CH | | 0.75 | | 0.75 | 1 | | 0.75 |

Symbols and abbreviations used in all tables herein:
Ex.: Example: n.a.: not applicable: n.av.: not available; n.d.: not determined; *: measured value differs from expected theoretical value due to method variability

The solid pharmaceutical formulation or dosage form for oral administration as obtained by the process as described herein is preferably in the form of a pellet or sphere, more preferably in the form of a micropellet or microsphere. All of the foregoing may be further incorporated into capsules, e.g. gelatine capsules: blisters, sachets or bottles, e g. PVC bottles, all suitable for pharmaceutical use.

Thus, in accordance with the above, the subsequent process steps of the process as described herein may comprise one or more of the following steps listed below:
- Cooling the extrudate obtained by the melt extrusion processes as described herein
- Optionally milling the extrudate and optionally screening the milled extrudate
- Cutting/breaking down the obtained extrudate to cylindrical pellets
- Spheronization of the extrudates to sphcrcs/microsphcres, pellets/micropellets or granules
- Drying
- Optionally (film) coating of the pellets, granules or granules
- Optionally blending the milled extrudate with one or more pharmaceutically acceptable excipients
- Optionally blending the pellets, spheres or granules with one or more pharmaceutically acceptable excipients
- Formulating the obtained blend into a solid oral dosage form such as a hard gelatine capsule or tablet or filling it into suitable containers like bottles, e.g. PVC bottles.

In a preferred embodiment, the processes as described herein comprise one or more of the above steps as continuous processes.

All processes as described above may be scaled-up as desired. For melt extrusion process variants as described herein it is known in the art that the basic geometry of the extruder should match the above described as closely as possible, while the ratio of the outer diameter (Dₒ) to the inner diameter (Dᵢ) of the screw is a key parameter. Moreover, also the screw profile and/or the screw bar configuration should be similar. However, as is known, mass and heat transfer limitations may arise, requiring the adjustment of the set up and longer process sections and/or alternative screw designs to ensure dispersion and uniformity (see e.g. Swanborough,A., "Benefits of Continuous Granulation for Pharmaceutical Research. Development and Manufacture", Application Note LR-63, Thermo Fisher Scientific, 2008 or Markarian, J., "Scale-up Challenges in Hot Melt Extrusion". Pharmaceutical Technology, 20 (4) (2012) 88-92)

In a further aspect, a pharmaceutical composition comprising a solid or semi-solid composition obtained by a process as described herein is provided, further optionally comprising one or more conventional pharmaceutically acceptable excipients. Pharmaceutical compositions provided by the process as described herein may be used to supplement digestive enzymes in the treatment and/or the prophylaxis of maldigestion in mammals, in particular of maldigestion due to chronic exocrine pancreatic insufficiency such as in patients suffering from cystic fibrosis, chronic pancreatitis or patients who have undergone upper gastrointestinal surgery. Pharmaceutical compositions or dosage forms as described herein may preferably be oral dosage forms which can in particular be administered to humans.

The solid or semi-solid compositions, and/or dosage forms as described herein may be further formulated into pharmaceutical compositions using one or more conventional pharmaceutically acceptable excipient(s) commonly used in formulation technology, e.g., such as inter alia referred to in Fiedler's "Lexikon der Hilfstoffe" 5th Edition, Editio Cantor Verlag Aulendorf 2002. "The Handbook of Pharmaceutical Excipients", 4th Edition, American Pharmaceuticals Association, 2003. and may be selected from carriers, diluents or fillers, binding agents, disintegrants, lubricants, glidants, stabilizing agents, surfactants, film-formers, softeners, wetting agents, sweeteners, pigments/colouring agents, antioxidants. and preservatives.

Suitable carriers, binding agents, disintegrants, lubricants and glidants can e.g be the ones described in more detail here above as pharmaceutically acceptable auxiliary agents. Furthermore, the pharmaceutical compositions and/or dosage forms as described herein may be coated employing film coatings or modified release coatings using known coating methods and commercially axailable coating materials such as a mixture of film-forming polymers, opacifiers, colorants and plasticizers. Preferably and due to their beneficial properties like excellent acid-stability. the pharmaceutical compositions and/or dosage forms as provided herein are preferably not coated with enteric coatings.

The dosage forms as described herein may he fonnulated in accordance with known methods, e.g., as described in "Pharmazeutische Technologic", 11th Edition Deutscher Apotheker Verlag 2010. or "Pharmazeutische Technologie", 9th Edition. Wissensclsnftliehe Verlagsgesellschaft Stuttgart, 2012.

Lists of further suitable excipients may also be found in textbooks such as Remington's Pharmaceutical Sciences, 18th Ed. (Alfonso R. Gennaro, ed.: Mack Publishing Company, Easton, PA, 1990); Remington; the Science and Practice of Pharmacy 19th Ed.( Lippincott, Williams & Wilkins. 1995); Handbook of Pharmaceutical Excipients, 3rd Ed. (Arthur H. Kibhe, ed.; Amer. Pharmaceutical Assoc. 1999); the Pharmaceutical Codex: Principles and Practice of Pharmaceutics 12th Ed. (Walter Lund ed, Pharmaceutical Press, London, 1994); The United States Pharmacopeia: The National Formulary (United States Pharmacopeial Convention); and Goodman and Gilman's: the Pharmacological Basis of Therapeutics (Louis S. Goodman and Lee E Limbird, eds.; McGraw Hill, 1992).

In preferred embodiments, spherical particles of a diameter of 0 5-2 mm (microspheres or micropellets) may be produced as described above which are then filled into hard gelatine capsules or bottles, without any further excipients or additives.

In a second aspect, the present invention also provides a solid or semi-solid composition, preferably for pharmaceutical use, comprising:
(a) 40-75 % by weight of the composition of pancreatin and, or a pancreatin containing mixture of digestive enzymes derived from a mammal:
(b) 10-50 % by weight of the composition of a surfactant-component of
   (i) 2-90 % by weight, relative to the surfactant-component, of at least one surfactant selected from (aa) non-ionic surfactants, comprising polyethylene glycol fatty acid mono- and/or di-esters with aliphatic C₆-C₂₂-carboxylic acids; polyethylene glycol glycerol fatty acid esters with aliphatic C₆-C₂₂-carboxylic acids; polyethylene glycol alkyl mono- and/or di-ethers with aliphatic C₁₂-C₁₈-alcohols. oligoethylene glycol ethers with aliphatic C₂-C₁₈-alcohols; and mixtures of any of the foregoing, and (bb) ionic surfactants, comprising lecithin, lysolecithin, phosphatidylcholine, phosphatidylethanolamine, phosphatidylglycerol, phosphatidyl serine, lysophosphatidylcholine, lysophosphatidylethanolamine, lysophosphatidylglycerol, lysophosphatidylinositol, lysophosphatidic acid, lysophosphatidylserine; and mixtures of any of the foregoing, and mixtures of any of the foregoing surfactants from (aa) and (bb);
   (ii) 5-60 % by weight, relative to the surfactant-component, of at least one co-surfactant selected from mono-acylglycerides with aliphatic C₆-C₂₂-carboxylic acids, mono-ethers of glycerol with aliphatic C₁₂-C₂₂-alcohols. partial esters of propylenglycol with aliphatic C₆-C₂₂-carboxylic acids, partial esters of polyglycerol with aliphatic C₆-C₂₂-carboxylic acids, oligoethylene glycol monoesters with aliphatic C₆-C₂₂-carboxylic acids, oligoethylene glycol diesters with aliphatic C₆-C₂₂-carboxylic acids and mixtures of any of the foregoing and
   (iii) 0-70 % by weight, relative to the surfactant-component, of a lipophilic phase selected from di- and triacylglycerides with aliphatic C₆-C₂₂-carboxylic acids and/or mixtures of any of the foregoing;
   wherein the percentages (i), (ii) and (iii) add to 100 % by weight for the surfactant-component in each case;
(c) one or more antioxidant(s) present in a concentration of 50 to 200 ppm
(d) 5-50 % by weight of a polymeric additive selected from hydrophilic polymers with melting points or glass transition temperatures of 50-160 °C;
whereby the ratio of the polymeric additive (d) and the surfactant-component (b) is 0.4 (2:5) to 1.5 (3:2), preferably 1 (1:1), and whereby all weight percentages of components (a), (b), (c) and (d) in the composition add to 100% by weight as claimed in appended claim 9.

The pancreatin and/or pancreatin containing mixture of digestive enzymes derived from a mammal (a) can preferably he present in an amount of 40-70 wt.-%, 45-68 wt.-%, 47-68 wt.-% or 68 wt.-% of the solid or semi-solid composition. In other particularly preferred embodiments, component (a) is present in an amount of 50-70 wt.-%, more preferred in an amount of 58-70 wt.-% (64 wt.-% +/- 6 wt -% ) and yet more preferred in an amount of 60-68 wt.-%.

The surfactant-component (b) can preferably be present in an amount of 15-40 wt.- %, 15-30 wt.- %. 15-25 wt.- %. 17.5-25 wt.- % or 20 wt - % of the solid or semi-solid composition. The components surfactant (i), co-surl'actant (ii) and lipophilic phase (iii) can preferably be present in the solid or semi-solid composition in the same amounts and ratios as described above for the processes of the first aspect of the invention.

The polymeric additive (d) can preferably be present in an amount of 5-35 wt.- %, 10-30 wt.-%, 10-25 wt.- % or 15-25 wt.- % of the solid or semi-solid composition.

The w/w-ratio between the polymeric additive component (d) and the surfactant-component (b) in the solid or semi-solid composition is between 0.4 (2:5) and 1.5 (3:2). preferably between 0.75 and 1.3.More preferably, their w/w-ratio is 1:1.

Component (a), the pancreatin and/or a pancreatin containing mixture of digestive enzymes derived from a mammal in the solid or semi-solid composition is preferably pancreatin and more preferably porcine pancreatin as generally used for therapeutic purposes, as explained herein in more detail.

Component (b), the surfactant-component can be the same or substantially the same in the solid or semi-solid composition as described above as preferred surfactant-components for the processes of the first aspect of the invention. Surfactant mixture (b) of the above solid or semi-solid composition can in particular and preferably be selected from Gelucire^{®} 44/14 and/or Gelucire^{®} 50/13, both as described above in detail. Solid or semi-solid compositions comprising Gelucire^{®} 44/14 as the surfactant mixture (b) are most preferred.

Component (c), is the same as
for the processes of the first aspect of the invention, i.e. one or more antioxidant(s), preferably BHA, in particular BHA, present in a concentration of
50 to 200 ppm, preferably of 100 to 150 ppm, in particular of 150 ppm, relative to the total weight of the solid or semi-solid composition.

Component (d), the polymeric additive can be the same or substantially the same in the solid or semi-solid composition as described above as suitable polymeric additives for the processes of the first aspect of the invention. Preferably, the polymeric additive is selected from hydrophilic polymers with melting points or glass transition temperatures of 50-70 °C. or of 50-65 °C. In other preferred embodiments, the polymeric additive is selected from hydrophilic polymers having a melting point or glass transition temperature of 50-110 °C. In one embodiment, said hydrophilic polymers with melting point or glass transition temperature of 50-110 °C are selected from polymers with a chain at least the ends of which are hydrophilic chains. In more preferred embodiments, the polymeric additive in the solid or semi-solid composition can be selected from PEGs and/or poloxamers which have an average molecular weight of 3000-30000 g/mol, more preferably of 3250-25000 g/mol. In particular, the polymeric additive in the solid or semi-solid composition can be PEG 4000, PEG 8000, PEG 20000 and/or poloxamer 188.

Preferred are moreover solid or semi-solid compositions comprising PEG 20000 as the hydrophilic polymer additive (d) at a (d):(h) ratio of 1:1 and compositions comprising PEG 4000 as the polymer additive (d), which is used in a ratio of (d):(b) of 1:1 to 2:1.5.

In aspects, the present invention also provides solid or semi-solid compositions comprising:
(a) an enzyme or enzyme mixture having lipase activity:
(b) a surfactant mixture of
   (i) at least one surfactant
   (ii) at least one co-surfactant and
   (iii) a lipophilic phase;
(c) one or more antioxidant(s) and
(d) a hydrophilic polymer additive with a melting point or glass transition temperature of 50-65 °C;
whereby the ratio of additive (d) and mixture (b) is 0.4 (2:5) to 1.5 (3:2) as claimed in appended claim 11.

Solid or semi-solid compositions as described herein in a second aspect of the invention may preferably he prepared by melt extrusion using a twin screw extruder, preferably a co-rotating twin screw extruder. In certain embodiments, said compositions can generally also be prepared at lower temperatures, e.g. at barrel temperatures of the extruder of about 50- 60 °C or at a product temperature of about 45-70 °C.

In a further embodiment the present invention also provides pharmaceutical compositions comprising solid or semi-solid composition according to the present invention and optionally further comprising one or more conventional pharmaceutically acceptable excipients. The conventional pharmaceutically acceptable excipients can be the same or substantially the same in the pharmaceutical composition as described above as suitable conventional pharmaceutically acceptable auxiliary agent(s) for the processes of the first aspect of the invention or may be selected from pharmaceutically acceptable auxiliary agent(s) as described above for the processes of the first aspect of the invention.

Exemplary formulations and processes for the preparation of the melt composition according to the process as described herein are detailed in the examples below. The following examples are therefore meant to illustrate the invention without limiting its scope.

### Examples

Pancreatin (porcine pancreatin powder, therapeutic grade) used in the examples below was provided by Abbott, Neustadt, Germany unless stated otherwise.

### 1. Melt Granulation

For manufacturing of the solid or semi-solid compositions by the process variant of melt granulation, surfactant-component (h) and/or polymeric additive (c) (as required by the target composition) were mixed in a glass beaker at the required weight ratios and heated (oil bath) until melting (usually at about 50 °C). One or more pharmaceutically acceptable auxiliary agent(s) (c) were added as required to the receiving melt so obtained (e.g. 150 ppm BHA. relative to the total weight of the receiving melt could be added to samples which were not foreseen for virus-spiking) and the combined components were mixed for about 5 min. with a spattle to achieve homogenization. Then, porcine pancreatin powder (virus-spiked or non-virus-spiked, as applicable), therapeutic grade, was added in the amount (weight ratio) desired to the receiving melt at about 50 °C and mixing was continued. Heating was carefully increased under stirring until the prescribed target (product) temperature of the mixture (70-130 °C), e.g. 120 °C was reached. Temperatures were measured with a digital inserting thermometer (Testo 720). After a defined holding time at the target temperature (e.g. 5 min.), samples of about 2 g were drawn from the glass beaker, at least one each from the top, middle and bottom of the glass beaker and passed through a sieve (800 micrometers). Samples (about 1 g) from non-virus-spiked porcine pancreatin were used for determining residual enzymatic activities. Similarly, samples (about 1 g) from virus-spiked pancreatin powder (without antioxidant/BHA) were used for determining residual viral load. The solid or semi-solid compositions as manufactured according to this protocol are shown in tables 2a and 2b, below.

Virus reduction factors (as Log Reduction Factor, LRV. as explained below) and recovery of lipase activity after the process run (measured as lipase activity found after the process run divided by lipase activity found before the process run, in %) were determined as explained in the methods section.

Examples 38-57 were produced by melt granulation processes according to the invention. Examples 38-43 and 47-57 were produced by preferred melt granulation processes according to the invention. Example Cl is a comparative example with a composition not according to the invention. Examples 36-54 and 56-57 represent solid or semi-solid compositions according to the invention.

**Table 2a: Illustrating compositions not covered by the present invention manufactured by melt granulation processes as described herein**

| **Composition(s)** | **Ex. 36** | **Ex. 37** | **Ex. 38** | **Ex. 39** | **Ex. 40** |
|---|---|---|---|---|---|
| Pancreatin [%] w/w (virus-spiked API) | 63.6 | 63.6 | 63.6 | 63.6 | 63.6 |
| Gelucire^{®} 44/14 [%] w/w | 18.2 | 18.2 | 18.2 | 18.2 | 18.2 |
| PEG 4000 [%] w/w | 18.2 | 18.2 | 18.2 | 18.2 | 18.2 |
| Product (target) temperature [°C] | 70 | 70 | 90 | 90 | 90 |
| Time product temperature applied [sec.] (hold time at target temperature) | 300 | 300 | 300 | 1800 | 300 |
| Virus type (spike) | PsRV | FCV | FCV | FCV | PPV |
| Virus reduction [LRV] | ≥ 6.8 | 1.7 | ≥ 6.37 | ≥ 6.37 | 0.94 |
| Virus reduction [LRV] for comparative study (pancreatin only) | ≥ 6.17 | 0.97 | ≥ 6.13 | ≥ 6.13 | 0.6 |

From the examples manufactured by melt granulation process variants as described herein it can be seen (cf. tables 2c-2c) that high to very high activities of target enzymes in porcine pancreatin can be preserved when process (target) temperatures of not less than 90°C and not exceeding 130 °C (90-130 °C). 95-125 °C or 105-130 °C are applied for time periods (process times: hold times at target temperatures) of not less than 30 sec. and not exceeding 45 min., like of not less than 180 see and not exceeding 30 min. or like of not less than 300 sec. and not exceeding 30 min.

**Table 2b: Illustrating compositions not covered by the present invention manufactured by melt granulation processes as described herein (continued)**

| **Composition(s)** | **Ex. 41** | **Ex. 42** | **Ex. 43** | **Ex. 44** | **Ex. 45** |
|---|---|---|---|---|---|
| Pancreatin [%] w/w (virus-spiked API) | 63.6 | 63.6 | 63.6 | 63.6 | 63.6 |
| Gelucire^{®} 44/14 [%] w/w | 18.2 | 18.2 | 18.2 | 18.2 | 18.2 |
| PEG 4000 [%] w/w | 18.2 | 18.2 | 18.2 | 18.2 | 18.2 |
| Product (target) temperature [°C] | 90 | 110 | 110 | 120 | 120 |
| Time product temperature applied [sec.] (hold time at target temperature) | 1800 | 300 | 1800 | 30 | 60 |
| Virus type (spike) | PPV | PPV | PPV | PPV | PPV |
| Virus reduction [LRV] | 2.15 | 2.14 | 3.24 | 1.0 | 1.6 |
| Virus reduction [LRV] for comparative study (pancreatin only) | 1.45 | 0.8 | 2.3 | n.d. | n.d. |

From the examples manufactured by melt granulation process variants as described herein it can also be seen (cf. tables 2a-2c) that the concentration of medium resistant viruses (using PsRV as a model for the type of medium resistant viruses) could be very significantly reduced in resulting solid or semi-solid compositions when processing the mixture at a temperature of 70 °C for a time period of 300 sec. (LRV ≥ 6.8). The concentration of medium to highly resistant viruses (using e.g. FCV as a model for the type of medium-highly resistant viruses) could be very significantly reduced in resulting solid compositions when processing the mixture at a temperature of 90 °C for a time period of 300 sec. (LRV ≥ 6.4). The concentration of highly resistant viruses (using e.g. PPV, as a model for the type of highly resistant viruses) could be significantly reduced (LRV about 3) in resulting solid compositions when processing the mixture at a temperature of 110 °C for a time period of 1800 sec. (30 min), or alternatively at a temperature of 120 °C for a time period of 180 sec. At a temperature of 120 °C for a time period of 300 sec., even significantly higher virus inactivation could be observed (LRV >/= 3.6).

**Table 2c: Illustrating compositions not covered by the present invention manufactured by melt granulation processes as described herein (continued)**

| **Coinposition(s)** | **Ex. 46** | **Ex. 47** | **Ex. 48** | **Ex. 49** |
|---|---|---|---|---|
| Pancreatin [%] w/w (virus-spiked API) | 63.6 | 63.6 | 63.6 | 63.6 |
| Gelucire^{®} 44/14 [%] w/w | 18.2 | 18.2 | 18.2 | 18.2 |
| PEG 4000 [%] w/w | 18.2 | 18.2 | 18.2 | 18.2 |
| Product (target) temperature [°C] | 120 | 120 | 120 | 120 |
| Time product temperature applied [sec.] (hold time at target temperature) | 150 | 180 | 300 | 1800 |
| Lipase activity recovered [%] | n.d. | n.d. | n.d. | n.d. |
| Protease activity recovered [%] | n.d. | n.d. | n.d. | n.d. |
| Amylase activity recovered [%] | n.d. | n.d. | n.d. | n.d. |
| Virus type (spike) | PPV | PPV | PPV | PPV |
| Virus reduction [LRV] | 2.2 | 2.94 | 3.64 | 4.6 |
| Virus reduction [LRV] for comparative study (pancreatin only) | n.d. | n.d. | 3.22 | 3.87 |

**Table 2d: Illustrating compositions not covered by the present invention manufactured by melt granulation processes as described herein (continued)**

| **Composition(s)** | **Ex. 50** | **Ex. 51** | **Ex. 52** | **Ex. S3** | **Ex. 54** |
|---|---|---|---|---|---|
| Pancreatin [%] w/w | 60 | 60 | 60 | 60 | 60 |
| Gelucire^{®} 44/14 [%] w/w | 20 | 20 | 20 | 20 | 20 |
| Vitamin E TPGS [%] w/w | 0 | 0 | 0 | 0 | 0 |
| PEG 4000 [%] w/w | 20 | 20 | 20 | 20 | 20 |
| Product (target) temperature [°C] | 120 | 120 | 130 | 130 | 110 |
| Time product temperature applied [sec.] (hold time at target temperature) | 300 | 1800 | 300 | 1800 | 1800 |
| Lipase activity recovered [%] | 100 | 94 | 91 | 74 | 102* |
| Protease activity recovered [%] | 105* | 104* | 99 | 93 | n.d. |
| Amylase activity recovered [%] | 89 | 81 | 63 | n.d. | n.d. |

Tables 2a-2c (last lines) also show, for comparison purposes, LRVs of preparations of pancreatin-only which had been treated in the same manner (virus type, spike, target temperature, hold time at target temperature) as the respective Example shown in the same row of the respective table. The data suggests that virus inactivation by heat application in pancreatin-only samples is significantly lower than virus inactivation in samples which were treated by a melt granulation processes variant according to the invention and/or in samples which represent solid or semi-solid compositions according to the invention.

**Table 2e: Illustrating compositions not covered by the present invention manufactured by melt granulation processes as described herein (continued)**

| **Composition(s)** | **Ex. 55** | **Ex. 56** | **Ex. 57** | **Ex. C1** |
|---|---|---|---|---|
| Pancreatin [%] w/w | 60 | 60 | 60 | 65 |
| Vitamin E TPGS [%] w/w | 20 | 0 | 0 | 0 |
| Labrafil^{™} M2125CS [%] w/w | 0 | 20 | 0 | 0 |
| Labrasol^{™} [%] w/w | 0 | 0 | 20 | 0 |
| PEG 4000 [%] w/w | 20 | 20 | 20 | 35 |
| Product (target) temperature [°C] | 105 | 110 | 110 | 110 |
| Time product temperature applied [sec.] (hold time at target temperature) | 300 | 600 | 600 | 1800 |
| Lipase activity recovered [%] | 92 | 105* | 104* | 96 |

### 2. Melt extrusion

To optimize the mixtures or compositions for usefulness in processes selected from melt pelletization, melt granulation and melt extrusion as described herein, in particular for melt extrusion, and to optimize the content of the API pancreatin. formulations with varying composition and API content were prepared and evaluated with regard to processibility in view of extrusion, spheronization and maintenance of the desired biological activity (lipase activity). Results from these experiments are summarized in tables 3a-3d, below. Lipase activities shown were determined as described in the methods section below.

Compositions as shown in tables 3a-3d were prepared by a melt extrusion process using a Gabler DE-40-T D15 twin-screw extruder (pilot-scale. screw diameter 40 mm. screw length 600 mm) with a die plate (1.0 mm wall thickness and 387 die holes of 1 .0 mm diameter, the die plate being fixed with a support plate to have 0.2-0.3 mm clearance to the extruder screws) and subsequent spheronization with a Gabler R 250 double jacket spheronizer fitted with a tempering device. The extruder screws had the features explained in the description of Fig. 1. Hereafter, reference signs and terminology of sections corresponding to those illustrated in Fig. 1 are used to indicate portions of the extruder.

Pancreatin powder or a premix with pancreatin was fed into the extruder and onto the solid substance feeding section 2 immediately after the initial section 1 using a loss in weight feeder.

The melt mass (components (b) and (d) of the preferred compositions shown above) was provided in a stirred vessel heated about 20 °C above its solidification point and was fed into the extruder onto the medium thread pitch section (melt feeding section) 3 immediately after the solid substance feeding section 2 using a pump (usually peristaltic dosage pump Verderflex, Verdcr, Haan, Germany). The pump's feeding tube was provided with an outer heating tube having a medium tempered at approximately 75 °C (+/-5 °C) and isolated against heat loss. Dosage of the melt was adjusted by setting the pump speed based on a mass-speed-calibration for the respective melt. Dosages of the melt and pancreatin were set according to the compositions shown in tables 3a-3c below.

In front of the die plate a compression results from the pressure of the materials conveyed by the extruder screws and the resistance of the die plate, so that a uniform extrudate is producable over the process period with homogenous composition and consistency. To establish this uniform extrudate, a steady flow of the melt from the heated vessel down through the die plate with the adjusted dosage of the melt was established first. Then the loss in weight feeder was operated according to a set pancreatin or pancreatin containing premix concentration in the extrudates to feed respective solid powders pancreatin onto a portion of the solid substance feeding section 2 of the extruder screws, which portion is adjacent to the interface between the initial section 1 and the solid substance feeding section 2. This processing sequence can avoid detrimental mechanical load. From a time when a steady extrudate composition was generated in the extruder and existed at the die plate until a time when the melt in the heated vessel or the pancreatin powder in the loss in weight feeder became short, a period was used for collecting extrudates using bowls made of stainless steel. This period was about I hour. Within this period, 8 kg of extrudates were produced in example "4" (cf. table 3a) while usually 10 kg of extrudates were produced in all other examples and comparative examples. After said period feeding of the pancreatin powder was stopped first and feeding of the melt was stopped thereafter.

During all operations of the extruder the barrel temperature of the extruder was set to about 50-60 °C (usually equivalent to product temperatures of about 45-70 °C). The first and second kneading element sections 4 and 6 served to mix the materials used for producing extrudates. The experiments in this section were run at barrel temperatures of below 90 °C (product temperatures not determined) but were found to be suitable for pre-selecting suitable mixtures and/or compositions for processing at higher temperatures (see below).

Spheronization of the extrudates, preferably to spherical pellet shapes. required establishing an inner extrudate temperature below the melting temperature of the extruded material, specifically sufficiently low to avoid agglomeration and sufficiently high to avoid an embrittlement which is detrimental for shaping, particularly sphcronizing.

In order to establish suitable inner extrudate temperatures, the collected extrudates were left at room temperature for 2 hours and then loaded into the spheronizer. Then the spheronizer was placed and operated in a heating cabinet at 50 °C in order to achieve a temperature of the material inside the spheronizer of close to 42 °C.

It was found that the spheronizer could be operated to produce uniform pellets at an average yield of 90 % within the following operating parameters per hatch: charge 250-400 g; spheronization period 4-7 min. and rotational speed 600-1200 rpm.

Classifications of the rounded pellets were performed using Krcssner sieves with with approx. 0.7 mm screen size and approx. 1.6 mm screen size, classifying yields were 75-80% of theoretical yield.

**Table 3a: Illustrating compositions not covered by the present invention manufactured by melt extrusion processes (pilot-scale) as described herein at ban-el temperatures of 50-60 °C.**

| **Composition(s)** | **Ex. 1** | **Ex. 2** | **Ex. 3** | **Ex. 4** | **Ex. 5** |
|---|---|---|---|---|---|
| Pancreatin [%] w/w | 60 | 60 | 60 | 66 | 60 |
| Gelucire^{®} 44/14 [%] w/w | 0 | 0 | 20 | 17 | 40 |
| Gelucire^{®} 55/13 [%] w/w | 40 | 20 | 0 | 0 | 0 |
| PEG 4000 [%] w/w | 0 | 20 | 20 | 17 | 0 |
| Lipase activity recovered [%] | 100 | 100 | 100 | 100 | 100 |
| Evaluation procossibility extrusion | (1) | (1) | (1) | (1) | (1) |
| Evaluation processibility spheronization | (01) | (01) | (01) | (01) | (01) |

Symbols used in tables 3a-3c for evaluation of extrusion (characterizing quality):
(1): no problems with extruding, good processibility; (2): processing possible, but high fraction > 1.6 mm (3): processing possible but not optimal, high viscosity; (4): too wet for processing; (5): very dry, high content of dust:

Symbols used in tables 3a-3c for evaluation of spheronization (characterizing quality):
(01): good-very good spheronization, low loss; (02) processing not optimal, build-up; (03) processing not optimal, low yield; (04): processing possible but not optimal.

Examples 2-4. 8-14, H and a-b represent solid or semi-solid compositions.

Examples 2, 3. 4, 8, 9, 10, 11, 12. 13, 14 and H represent preferred solid or semi-solid compositions. Examples shown in tables 3a-3c were produced by melt extrusion processes similar to the ones according to the invention.

**Table 3b: Illustrating compositions not covered by the present invention manufactured by melt extrusion processes (pilot-scale) as described herein at barrel temperatures of 50-60 °C (continued)**

| **Composition(s)** | **Ex. 6** | **Ex. 7** | **Ex. 8** | **Ex. 9** | **Ex. 10** |
|---|---|---|---|---|---|
| Pancreatin [%] w/w | 62 | 60 | 60 | 60 | 60 |
| Gelucire^{®} 44/14 [%] w/w | 38 | 35 | 20 | 17.5 | 25 |
| Gelucire^{®} 55/13 [%] w/w | 0 | 0 | 0 | 0 | 0 |
| Labrasol^{™} [%] w/w | 0 | 5 | 0 | 0 | 0 |
| Lecithin [%] w/w | 0 | 0 | 0 | 5 | 0 |
| PEG 4000 [%] w/w | 0 | 0 | 0 | 17.5 | 0 |
| PEG 20000 [%] w/w | 0 | 0 | 20 | 0 | 0 |
| Poloxamer 188 [%] w/w | 0 | 0 | 0 | 0 | 15 |
| Lipase activity recovered [%] | 100 | 100 | 100 | 100 | 100 |
| Evaluation processibility extrusion | (1) | (1) | (1) | (1) | (1) |
| Evaluation processibilily spheronization | (01) | (01) | (01) | (01) | (01) |

From the experiments shown in tables 3a-3c it can be estimated that mixtures for the preparation of solid or semi-solid compositions with a content of pancreatin of about 40-75 wt.-% can be smoothly processed in melt-extrusion processes as described herein when the components and their amounts and ratios are selected according to the present disclosure and that the solid or semi-solid compositions resulting from such processes have preserved desired enzymatic activities to the highest extent. Other I processes and solid or semi-solid compositions can be obtained when selecting the process variants and compositions characterized herein as preferred or more preferred.

**Table 3c:Illustrating compositions not covered by the present invention manufactured by melt extrusion processes (pilot-scale) as described herein at barrel temperatures of 50-60 °C (continued)**

| **Composition(s)** | **Ex. 11** | **Ex. 12** | **Ex. 13** | **Ex. 14** | **Ex. H** |
|---|---|---|---|---|---|
| Pancreatin [%] w/w | 60 | 60 | 47 | 47 | 60 |
| Gelucire^{®} 44/14 [%] w/w | 20 | 20 | 15 | 15 | 15 |
| Labrasol^{™} [%] w/w | 0 | 0 | 0 | 0 | 5 |
| PEG 4000 [%] w/w | 15 | 10 | 15 | 15 | 20 |
| Crospovidon [%] w/w | 5 | 10 | 0 | 0 | 0 |
| Microcrystalline cellulose MCC 101 [%] w/w | 0 | 0 | 23 | 0 | 0 |
| Sucrose [%] w/w | 0 | 0 | 0 | 23 | 0 |
| Lipase activity recovered [%] | 100 | 100 | 100 | 100 | 100 |
| Evaluation processibility extrusion | (1) | (1) | (1) | (1) | (1) |
| Evaluation processibility spheronization | (01) | (01) | (01) | (01) | (01) |

Thus in one embodiment , solid or semi-solid compositions comprise PEG 20000 as the polymer additive (d) and having a (d):(b) ratio of 1:1 and compositions comprising PEG 4000 as the polymer additive (d), which is used in a ratio of (d):(b) of 1: 1 to 2:1.5.

In further experiments, solid or semi-solid compositions were produced by twin-screw melt extrusion processes on different scales. A general protocol for such processes is provided below. The extruders mentioned therein servo to illustrate certain aspects of the invention without limiting its scope, while they, on the other hand, represent preferred embodiments for conducting a twin screw melt extrusion process as described herein, according to a first aspect of the invention:

**Table 3d: Illustrating compositions not covered by the present invention manufactured by melt extrusion processes (pilot-scale) as described herein at barrel temperatures of 50-60 °C (continued)**

| **Composition(s)** | **Ex. a** | **Ex. b** | **Ex. c** | **Ex. d** | **Ex. f** | **Ex. g** |
|---|---|---|---|---|---|---|
| Pancreatin [%] w/w | 50 | 55 | 70 | 68 | 60 | 60 |
| Gelucire^{®} 44/14 [%] w/w | 10 | 10 | 30 | 32 | 20 | 17.5 |
| Labrafil^{™} M2125CS [%] w/w | 0 | 0 | 0 | 0 | 0 | 5 |
| PEG 4000 [%] w/w | 40 | 35 | 0 | 0 | 0 | 0 |
| PEG 1500 [%] w/w | 0 | 0 | 0 | 0 | 20 | 17.5 |
| Lipase activity recovered [%] | n.d. | n.d. | 99 | 97 | n.d. | 100 |
| Evaluation processibility extrusion | (4) | (4) | (5) | (3) | (4) | (2) |
| Evaluation processibility spheronization | n.d. | n.d. | (02) | (03) | n.d. | (04) |

The melted mass is prepared by mixing and heating surfactant-component (b) (e.g. Gelucirc^{®} 44/14) and/or polymeric additive (d) (e.g. PEG 4000) in the w/w ratios required to arrive at the desired composition (e.g. at a ratio of 1:1 w/w), prior to the extrusion process. One or more pharmaceutically acceptable auxiliary agent(s) (c) are added in the required amount/ratio (e.g. 150 ppm BHA. relative to the total weight of the melted mass before the heating and mixing starts. The melted mass is then passed into a glass beaker filled with a magnetic stirrer and is heated under continuous stirring to a suitable temperature to avoid solidification of the melted mass during the process (e.g. about 75 +/- 5 °C) for the entire duration of the manufacturing process. A pipe system (heated to about 80 °C) transports the melted mass into the extruder via a suitable pump, preferably a heated pump (e.g. a heated HNP pump). Before the API (pancreatin powder) is fed into the extruder, the melted mass is passed onto the screw bars of the extruder by a pour hole (the dosing of the melted mass is thus carried out by a heated pump system and a heated pipe). The composition of the respective formulation is controlled by the weigh-in process, as well as by adjusting the feeding rate of the pancreatin powder (solid feeding system) and by the feeding rate of the melted mass which is monitored by the pump unit. During a pre-setting phase, the process settings and the composition of the formulations are adjusted for the respective excipients.

Before the extrusion process is started for both feeding devices, the solid feeder (for the API powder, e.g. the ZD 5 gravimetric solid feeder) as well as the melt feeder (for the melted mass) are calibrated by compiling characteristic dosing control curves. Similarly, the liquid feeding unit (HNP pump) is calibrated. Finally, a weight check for the output rates which were adjusted in order to obtain the required ratio of pancreatin: melted mass is performed Once the set points for the feeding rates of both components (API and melted mass) are confirmed to be in accordance with the formulation of the composition, the extrusion run is started.

The API (pancreatin) is added as a solid powder on the screw bars of the extruder using a feeder system (e.g. K-Tron feeder for pilot scale; Three Tec 5 mm feeder for miniature scale), whereby the feeding rate is controlled gravimetrically by the feeding system.

The barrels of the extruder are heated to the target temperatures (suitable to arrive at the required product temperatures) prior to starting the extrusion and the dosing of the components (incl. optional excipients). At die end of the extruder, the melt composition is passed through a die plate (die hole diameter about 1.0 mm) to produce several "spaghetti"-like extrudate strands. During the extrusion process, the throughput rate is controlled by weighing the extrudate and the consumed amount of melted mass over a certain time period (about 10 min). For process run times (per batch) of about 40 min., batch sizes result of about 100 g for the miniature scale and of about 5.3 kg for the pilot scale (batch size with regard to the effective output/yield).

After cooling to room temperature, a suitable portion of the extrudate so received is transferred into a common spheronizer (e.g. Gabler Spheronizer R-250) which is pre-heated to a suitable temperature, e g. about 49 °C. The extrudate is then rounded off to give approximately spherically shaped pellets with diameters of about 2 mm. Alter cooling to room temperature, the pancreatin pellets are graded with a 0.7 mm Kressner sieve (undersize grain) and then with a 1.6 mm Kressner sieve (oversize grain) to yield about 90 % of theoretical yield pancreatin pellets in a representative process run. The finished pancreatin pellets can then be filled into suitable containers (e.g. PVC bottles) for storage.

Minimal residence time of an extrusion run is determined by visual inspection using a suitable tracer or marker substance (e.g. curcumin from Merck Darmstadt, Germany). After stabilization of the processing conditions, one single dose of marker substance (curcumin) is added punctually to the pancreatin powder (about 50 mg curcumin at a throughput rate of 2.5 g/min for miniature scale, about 500 mg curcumin at a throughput rate of 8 kg/h for pilot scale) and the so marked pancreatin is fed directly into the inlet port of the extruder. The time period until the marked pancreatin first appears at the die end of the extruder is then recorded as minimal residence time by visual inspection.

In an alternative process, minimal residence time can also be determined by spectroscopy. Pancreatin powder marked with e.g. curcumin is fed directly into the inlet port of the extruder as described above. From the running extrusion process, samples are drawn in 5 sec. intervals (about 0.2 g each for miniature scale, about 2 g each for pilot scale) after about 55 sec. and over a time period of about 150 sec. Samples (about 7.3 g per sample) are then dissolved in accton (about 66 ml per sample) and the concentration of cucurmin in the samples is determined photometrically at a wavelength of λ=421 nm (Shimadzu UV-1602). The concentration values are plotted against time scale to give the concentration vs. time curve. In this method, it is not needed to determine the absolute concentration or amount of the tracer (e.g. cucurmin) as only a relative comparison between samples drawn at different times is needed. For finding the minimal residence time it is sufficient to determine the onset of the residence time distribution curve representing the initial appearance of the tracer at the die side.

A comparison of the minimal residence times found by the visual inspection method and the spectroscopy method, respectively, for selected process runs is provided in table 4, showing a very good correlation of results obtained from both methods for one given process run.

Preferred compositions (melt compositions or solid compositions) suitable For use with the processes as described herein, in particular for melt extrusion process variants, are shown in table 5 below.

**Table 4: Process parameters of exemplary extrusion processes for the process as described herein (different scales, twin-screw extrusion)**

| **Extruder model/type** | Gabler DE-40-T (40 mm) | Gabler DE-40-T (40 mm | ZE-9 (9mm) | ZE-9 (9mm) |
|---|---|---|---|---|
| **Screw bar configuration** | X4.1 | X4.1 | X4.1 | X4.1 |
| **Temp extrusion[°C] (barrel inlet)** | 108.3 | 109.5 | 114 | 113 |
| **Range of maximal extrudate temp. [°C]** | 107-112 | 105-110 | 104-107 | 103-105 |
| **Average maximal extrudate temp. [°C]** | 108.4 | 107.9 | 105.9 | 104.2 |
| **Screw speed (rpm)** | 72 | 72 | 62 | 62 |
| **Throughput rate [kg/h]** | 8 | 8 | | |
| **Average throughput rate [g/min]** | 133.5 | 132.5 | 2.5 | 2.5 |
| **Min. residence time (visual inspection), [sec.]** | 83 | 84 | 85 | 85 |
| **Min. residence time (curcumin distribution), [sec.]** | 85 (80-85) | 89 (85-90) | 85 (80-85) | 88 (85-90) |
| **Range of min residence time[sec.]** | 83-89 | | 85-88 | |

**Table 5: Solid or semi-solid compositions for use in the processes as described herein**

| **Composition(s)** | **Ex. 15** | **Ex. 16** | **Ex. 17** | **Ex. 18** |
|---|---|---|---|---|
| Pancreatin [%] w/w | 61.3 | 64.4 | 64.6 | 65.2 |
| Gelucire^{®} 44/14 [%] w/w | 19.4 | 17.8 | 17.7 | 17.4 |
| PEG 4000 [%] w/w | 19.4 | 17.8 | 17.7 | 17.4 |
| BHA [%] w/w | 150 ppm | 150 ppm | 150 ppm | 150 ppm |

In table 6, exemplary formulations and process parameters are summarized for melt extrusion process variants of the processes as described herein which use twin-screw extrusion at the miniature scale (Three Tec extruder ZE9) and certain screw bar configurations.

**Table 6a: Exemplary illustrating formulations not covered by the present invention and process parameters for melt extrusion processes as described herein (screw bar set up "X4.1" or modifications thereof)**

| **Composition(s)** | **Ex.19** | **Ex. 20** | **Ex. 21** | **Ex. 22** | **(Ex. 23)** |
|---|---|---|---|---|---|
| Pancreatin [%] w/w | 60 | 62 | 62 | 62 | 62 |
| Gelucire 44/14 [%] w/w | 20 | 19 | 19 | 19 | 19 |
| PEG 4,000 [%] w/w | 20 | 19 | 19 | 19 | 19 |
| Minimal Residence Time [sec.]¹ | 64 | n.d. | 69 | 69 | n.d. |
| Screw speed [rpm] | 75 | 75 | 75 | 75 | 65 |
| Throughput rate² [g/min] | 3.98 | 3.93 | 3.9 | 3.9 | 3.65 |
| Barrel temperature [°C] | 115 | 115 | 110 | 115 | 130 |
| Temp at die [°C] | 108 | 107 | 103 | 105 | 119 |
| Lipase activity recovered | 101.2 | 97.3 | 102.5 | 96.8 | 55 |
| Amylase activity recovered [%] | 93.5 | 87.8 | 93.7 | 85.7 | 20.2 |

Examples 19-24 represent solid or semi-solid compositions and were produced by melt extrusion processes according to the invention. Process run for example 23 was defective as target product temperature of 130 °C was exceeded due to overheating and congestion at the pancreatin powder inlet. Examples C2-C4 are comparative examples which do not represent solid or semi-solid compositions according to the invention but examples C3 and C4 were produced by melt extrusion processes according to the invention. Although examples C3-C4 could be processed, the resulting extrudates/strands were not optimal for further processing into stable pharmaceutical products as the consistence of the strands obtained was too soft and sticky. Comparison of processibility / results for Ex. 5 and 6 with Ex. C3 and C4 suggests that solid or semi-solid compositions according to the invention show superior processibility under conditions of the processes according to the invention (comprising a treatment at higher temperatures), in particular in melt extrusion process variants.

**Table 6b:Exemplary illustrating formulations not covered by the present invention and process parameters for melt extrusion processes as described herein (screw bar set up "X4.1" or modifications thereof), continued**

| **Composition(s)** | **Ex. 24** | **Ex. 25** | **Ex. C2** | **Ex C3** | **Ex. C4** |
|---|---|---|---|---|---|
| Pancreatin [%] w/w | 64 | 64 | 65 | 60 | 62 |
| Gelucire 44/14 [%] w/w | 18 | 18 | 0 | 40 | 38 |
| PEG 4,000 [%] w/w | 18 | 18 | 35 | 0 | 0 |
| Minimal Residence Time [sec.]¹ | n.d. | 70 | 80³ | 80³ | 80³ |
| Screw speed [rpm] | 70 | 70 | 70 | 70 | 70 |
| Throughput rate² [g/min] | 3.68 | 2.3 | 2.5 | 2.5 | 2.5 |
| Barrel temperature [°C] | 115 | 120 | n.av. | n.av. | n.av. |
| Temp at die [°C] | 107 | 112 | 108 | 100 | 100 |
| Lipase activity recovered [%] | 100.7 | 91.3 | 54 | n.d. | n.d. |
| Amylose activity recovered [%] | 90.6 | 76.4 | n.av. | n.d. | n.d. |

Explanation of footnotes used in tables 6a and 6b: ¹: by visual inspection; ²: by output weight ³: Minimal Residence Time not determined but estimated from other process runs with similar parameters

In table 7, exemplary formulations and process parameters are summarized for melt extrusion process variants of the processes as described herein which use twin-screw extrusion at the laboratory scale and certain screw bar configurations.

**Table 7: Exemplary formulations and process parameters for melt extrusion processes as described herein**

| **Composition** | **Ex. 26*** | **Ex. 27*** | **Ex. 28*** | **Ex. 29** | **Ex. 30** | **Ex. 31** |
|---|---|---|---|---|---|---|
| Pancreatin [%] w/w | 60.35 | 61.4 | 61.2 | 65 | 62 | 62 |
| Gelucire^{®} 44/14 [%] w/w | 19.825 | 19.3 | 19.4 | 17.5 | 19 | 19 |
| PEG 4000 [%] w/w | 19.825 | 19.3 | 19.4 | 17.5 | 19 | 19 |
| BHA [ppm] | 0 | 0 | 0 | 150 | 150 | 150 |
| Minimal Residence Time [sec.] | n.d. | n.d. | n.d. | n.d. | 82 | n.d. |
| Screw bar set up | X4.1 | n.d. | B2# | X4.1 | X4.1 (mod.) | X4.1 (mod.) |
| Screw speed [rpm] | 70 | 85 | 85 | 72 | 65 | 80 |
| Throughput rate [kg/h] | 7.655 | 7.8 | 7.8 | 8.01 | 8 | 8 |
| Barrel temp [°C] | 97 | 97.1 | 108.2 | 106.9 | Ca. 100 | Ca. 97 |
| Temp at die [°C] | 113 | 100.2 | 120.2 | 108.6 | 118.5 | 102.7-115.5 |
| Lipase activity recovered [%] | 103.2 | 100.7 | 90.9 | n.d. | 103.2 | 104.8 |
| Protease activity recovered [%] | n.d. | n.d. | n.d. | n.d. | n.d. | 102.5 |
| Amylase activity recovered [%] | 98.4 | 99.9 | 77.0 | n.d. | n.d. | 101.6 |

Explanation of footnotes used in table 7: mod.: modified: # B2: screw bar set-up similar to X4.1 but applying more mechanical stress
* illustrating formulations not covered by the present invention

In table 8, exemplary formulations and process parameters are summarized for melt extrusion process variants of the processes as described herein which use twin-screw extrusion at the pilot scale (Gabler DE 40 extruder) and certain screw bar configurations. Pancreatin in Ex. 58-60 was supplied by SPL.

**Table 8: Exemplary illustrating formulations not covered by the present invention and process parameters for melt extrusion processes as described herein (screw bar set up "X4.1 ")**

| **Composition** | **Ex. 58** | **Ex. 59** | **Ex. 60** |
|---|---|---|---|
| Pancreatin [%] w/w | 76 | 73 | 74 |
| Gelucire^{®} 44/14 [%] w/w | 12 | 13.5 | 13 |
| PEG 4000 [%] w/w | 12 | 13.5 | 13 |
| Minimal Residence Time [sec.] | Ca. 70-80 | Ca. 70-80 | Ca. 70-80 |
| Screw speed [rpm] | 75 | 75 | 75 |
| Throughput rate [kg/h] | 8 | 8 | 8 |
| Barrel temp [°C] | 105 | 96 | 90 |
| Temp at die [°C] | 106-108 | 108-110 | 107-110 |
| Lipase activity recovered [%] | 88.7 | 101.6 | 99.9 |
| Protease activity recovered [%] | 86.4 | 96.5 | 92.5 |
| Evaluation processibility extrusion | (1) | (1) | (1) |
| Evaluation processibility spheronization | (02) | (01) | (01) |

Symbols used in table 8 for evaluation of extrusion (characterizing quality): (1): good processibility. Symbols used in table 8 for evaluation of spheronization (characterizing quality): (01): good spheronization, low loss, (02) spheronization possible, but lower yield of rounded pellets.

From the experimental examples provided herein it could also been seen that processing times at product temperatures to effectively reduce viral loads in a biological material derived from a tissue of human or mammalian animal origin, in particular in pancreatin, could be further reduced in preferred process variants using a homogenizing extruder, to time periods of e.g. less than 10 min. like to less than 5 min.

### 3. Comparative Tests

### Influence of extrusion on virus inactivation

A conventional extrusion process with subsequent drying step was studied for its potential to reduce the infectious viral load in a pancreatin sample.

Pancreatin intermediate from production process (therapeutic grade) was spiked with FCV high-titer virus suspension similar as described in the methods section with the following deviations: stock solution was used "as is", liquid was filtrated off the spiked sample to receive a material equivalent to the moist process intermediate before drying. Drying was performed in lab scale equipment resulting in spiked pancreatin. Pancreatin was gently milled with a pestle and re-moistured with pure isopropanol (50% w/w relative to the pancreatin used). A pre-treatment sample was drawn to determine total virus load as described in the methods section. The moistened pancreatin was fed to a conventional single-screw extruder (type: Wyss& Probst Pharmcs 35T) equipped with holes (diameter 1.0 mm) in a plate (thickness 0.8 mm) and twice extruded at a screw speed of 45 rpm. After extrusion (about 90 min. in total) a post-treatment sample was drawn and analyzed for virus content. Subsequently, the extruded pancreatin was aliquoted into glass bottles (about 2.5 g each) and sealed. Immediately before the vials were put into a pre-heated cabinet drier (45°C with temperature monitoring), seals were removed. Samples from the drier were drawn after 90 min.. 12h, 20h and 60h and analyzed for viral content in each case similar as described in the methods section.

Data from this experiment (see table 9) suggests that extrusion and/or heating to 45 °C has no effect on virus inactivation in wetted pancreatin.

**Table 9: Influence of conventional extrusion process on viral reduction in a wetted pancreatin sample**

| **Sample (pancreatin)** | **Log₁₀ total virus load** | **LRV** |
|---|---|---|
| After wetting with isopropanol (load) | 8.41 | --- |
| After 2^{nd} extrusion | 8.11 | 0.30*¹ |
| After 90 min. drying at 45 °C | 7.39 | 0.72*² |
| After 12 h drying at 45 °C | 8.88 | - 0.77*² |
| After 20 h drying at 45 °C | 8.35 | - 0.24*² |
| After 60 h drying at 45 °C | 8.40 | - 0.29*² |

| | | |
|---|---|---|
| ¹: refers to load sample after wetting; ²: refers to sample after second extrusion. | | |

### Influence of surfactant-component on virus inactivation

It is known from the prior art that the use of detergents can help to inactivate enveloped viruses in biological material (see e.g. Soler. "Biopharm Int. (2002). 15(9), 28 42) while non-enveloped viruses can usually not be inactivated by detergents (see e.g. WHO Technical Report, Series No. 924, 2004, Annex 4, p. 168). In own experiments it could be confirmed that Gelucire^{®} 44/14 and Gclucire^{®} 55/13 alone at 37 °C only showed moderate inactivation of enveloped viruses (LRV 2-3; bovine diarrhea virus, PsRV) while no inactivation of non-enveloped viruses (rotavirus group A, FCV, PPV) was observed under these conditions.

### Methods

### 1. General method for evaluation of viral clearance with spiked preparations

For determining viral reduction as a consequence of the applied process variant, samples of component (a) (API, pancreatin) were spiked with a defined concentration of suitable model virus before the process and the remaining virus concentration after the process run was analyzed, as described in more detail below. Similarly, but in different samples which were not spiked with viruses, lead enzymatic activities of the API (pancreatin) were determined before the process and after the process run, as described in more detail below.

*Process for determining reduction of* viral *load* - *VCS (Viral Clearance Study)* Demonstration of virus inactivation or removal capability of biopharmaceutical manufacturing processes was done by deliberate spiking of a down-scaled version of the specific manufacturing process step with high-titer preparations of model viruses (e.g. *Pseudorahies virus*. "PsRV", an enveloped DNA-virus, low resistant). *feline Calicivirus* ("FCV"). a non-enveloped RNA-virus (medium-highly resistant). *Reovirus Type* 3 (Reo3), a non-enveloped RNA-virus (medium to highly resistant) and *porcine Parvovirus* PPV, a non-enveloped DNA-virus (very highly resistant) and subsequent comparison of the pre-treatment and post-treatment samples' infectious virus titers in cell-culture based, virus-specific infectivity assays. Parvoviruses represent viruses of greatest interest in preparations intended for use in humans and/or other animals, as they are ubiquitous in swine and represent and model the most difficult target contaminants to inactivate, given the sensitivity of the active pharmaceutical ingredient Accordingly, demonstration that a target parvovirus has been inactivated is usually accepted as evidence that the disclosed method will also inactivate other similar or less resistant viruses, e.g., non-enveloped viruses and larger enveloped viruses.

Examples described herein were performed according to recommendations of the "Note for guidance on virus validation studies: The design, contribution and interpretation of studies validating the inactivation and removal of viruses (CPMP/BWP/268195, February 1996)".

The starting material of each process was pancreatin powder (API) spiked with the target model virus of interest (e.g. PPV). High-titer virus stock suspensions were harvested from cell-culture, freeze-dried in a lyophilization chamber and virus titer (infectivity) checked in a sub-fraction of the lyophilisate. Shortly before the viral clearance experiments, the virus lyophilisate was homogeneously mixed with drug substance by a milling process to achieve a virus spike to API ratio of approximately 5-10 % (w/w). The spiked API was then used for the melt extrusion or melt granulation processes, as applicable.

As recognized in the art and as used herein, reduction in the viral titer when comparing two samples is normally reported as "log reduction value" ("LRV"), also sometimes referred to as "log reduction factor". A log reduction indicates the reduction in virus concentration in logarithmic units of base 10. For example, a log titer reduction with an LRV of 1 indicates a reduction of viral concentration by 90 % (i.e. number of viruses found after the process is 10 times smaller than before the process was applied); a log titer reduction with an LRV of 2 indicates a reduction of viral concentration by 99 % (i.e. number of viruses found after the process is 100 times smaller than before the process was applied); a log titer reduction with an LRV of 3 indicates a reduction of viral concentration by 99.9 % (i.e. number of viruses found after the process is 1000 times smaller than before the process was applied), and a log titer reduction with an LRV of 4 indicates a reduction of viral concentration by 99.99 % (i.e. number of viruses found after the process is 10000 times smaller than before the process was applied).

During the viral clearance experiments, process samples were withdrawn directly before the treatment and after the virus inactivation treatment (extrusion process or melt granulation process, as applicable) and quantitatively analyzed by standard endpoint titration and for the post-treatment samples in addition by large volume plating.

Generally, for each model virus, a corresponding system of a permissive cell-line and virus strain was used to determine the infectivity before and after treatment. The virus stocks were prepared from master virus stocks characterized e.g. by sequence analysis, growth kinetics, phenotypic analysis and/or by immunostaining with the corresponding antibody (e.g. anti-PPV). The master virus stocks were used to prepare intermediate virus stock, and derived thereof virus working lots were prepared which served, after lyophilisation, as virus spike preparation. The cell cultures employed in the virus clearance experiments were derived from master cell hanks, characterized by e.g. identity, purity and absence of mycoplasmas. The master cell banks were used to prepare the working cell banks. For this, cells were sub-cultured in cell culture flasks at a cell-line specific density and after reaching confluence or high cell density, harvested, centrifuged, counted, and seeded in fresh cell culture flasks and/or micro titer plates at cell specific density for the titration of the pre- and post-treatment samples. Matching systems of model virus strain and permissive cell line were as follows: PsRV-strain AK MK 35 in Vero 76 cells, an African green monkey kidney cell line; FCV-strain F9 (ATCC VR-782) in KE-R CCLV-RIE 138. a feline embryonic cell line; PPV-strain NADL-2 (ATCC VR-742) in SK6 CCLV-RIE 262, a porcine kidney cell line; Reo3- strain RVB-011 in HEK 293, a human embryonic kidney cell line. Cell lines "Vero 76", "KE-R CCLV-RIE 138".

"SKIS CCLV-RIE 262". and viral strains "PsRV AK MK35" and "Reo-3 RVB-011" can be obtained from the cell bank of the FLI (Friedrich-Löffler-Institut) Bundcsforschungsinstitut für Tiergesundheit, Südufer 10, 17493 Greifswald. Insel Riems, Germany, also see the FLI's website at: https://www.fli.bund.de/de/startseitc/seivice. Cell line "HEK 293" (CRL-1573) and viral strains "FCV Γ9" (VR-782) and "PPV NADL-2" (VR-742) can be obtained from the "American Type Culture Collection" (ATCC) at: LGC Standards GmbH, Mercatorstr. 51. 46485 Wesel, also see the ATCC's website at http://www.lgestandards-atce.org/products/all.

In addition, prior to the actual viral clearance experiments the non-interfering and non-cytotoxic sample dilution had been determined by cytotoxicity and interference pre-testing for the starting material (un-spiked API, representative for pre-traatment samples) and the formulated API (melt extrudate or melt granulate, as applicable, representative for post-treatment samples).

Virus tilers were determined by endpoint titration and large volume plating. For end-point titration, serial three-fold dilutions were prepared from the non-interfering and non-cytotoxic concentration of the sample with cell-culture medium and cultivated on the respective detector cell-line for a specified incubation period. The read-out was done by microscopic inspection for the virus-induced changes in cell-morphology (cytopathogenic effects. CPE) and for PPV in addition by immune-staining. For large volume plating, a greater volume of the re-suspended non-interfering and non-cytotoxic concentration of the test article was added to a defined number of wells containing the respective detector cell-line in cell culture-medium. The read-out was performed analogously to the endpoint titration method.

For the calculation of the half maximum tissue infection dose value (TCID₅₀-value), its standard deviation and calculation of the 95% confidence limits, the Spearman-Kaerber algorithm as given by Loewer was applied (see Bundesanzeiger Nr. 84, S. 3-8, 1994). In the case that no virus could be found in all parallels of the lowest sample dilution or in large volume plating, the titre was calculated by the Poisson formula and given as log₁₀ TCID50/ml with 95% probability. Reduction factors were determined as differences of log₁₀ TCID50-values of the pre-treatment samples (load samples before extrusion) and post-treatment samples (i.e., after extrusion).

### Melt extrusion

A miniature melt extruder (Three Tee extruder ZE 9) was used to reproduce relevant melt extrusion process parameters. Preparation of the melted mass of excipients was performed as described for the formulation experiments. API was homogenously spiked with a lyophilized model virus preparation to achieve an, c.g., 5-10 % spike (w/w). The dosing of the melted mass and feeding of the spiked API was realized as described for the formulation experiments. Once the set points for the feeding rates were confirmed to control the formulation composition, the extrusion run was started. Samples for analysis of the virus titer were taken just before the spiked API was filled into the solid feeding unit and immediately diluted for titration on the respective indicator cells. The processed material was released as extrudate from the mini-extruder through the dye plate and collected in a glass beaker. After a stable state of the extrusion process had been achieved (about 15 min.), extrudate for the post-treatment process sample was collected. After approximately 10 g had been collected, the collected extrudate was cooled to ambient temperature. Then the extrudate was processed through a sieve (800 micrometers) and two times 0.5 g of the powder were weighed out, re-suspended in cell-culture medium to the pre-determined non-cytotoxic and non-interfering concentration and immediately titrated on the respective indicator cells.

Process parameters applied for four manufacturing samples by twin-screw melt extrusion on miniature scale are shown in tables 10 and 11 below. Examples 32-35 contained wt.-% API as shown in table 11. Each composition 32-35 further contained Gelucire^{®} 44/14 and PEG 1:1 (w/w) to make up to 100 wt.-% and 150 ppm BHA. API (porcine pancreatin) from the same batch was used for preparing all four example compositions. Results from viral analytics of the twin-screw melt extruded samples are shown in table 11 below.

**Table 10: Process parameters for manufacturing samples (extrudates) by twin-screw melt extrusion for viral analytics**

| **Example** | **Screw bar set up** | **Virus** | **Screw speed [rpm]** | **Product temperature. [°C]** | **Throughput [g/min]** |
|---|---|---|---|---|---|
| Ex. 32 | X4.1 | Reo3 | 70 | 103 | 2.4 |
| Ex. 33 | X4.1 | PsRV | 65 | 105 | 2.5 |
| Ex. 34 | X4.1 | PPV | 62 | 106 | 2.6 |
| Ex. 35 | X4.1 | FCV | 62 | 107 | 2.6 |

**Table 11: Samples (extrudates) manufactured by twin-screw melt extrusion on miniature scale for viral analytics and summary of results for the virus inactivation capability of the melt extrusion process**

| **Example** | **Virus** | **Product temp. [°C]** | **Spike [%]** | **Content of spiked API [wt.-%]** | **LRV [log₁₀ reduction value]** |
|---|---|---|---|---|---|
| Ex. 32 | Reo3 | 103 | 5 | 60.9 | ≥ 6.2 # |
| Ex. 33 | PsRV | 105 | 5 | 60.4 | ≥ 3.5 # |
| Ex. 34 | PPV | 106 | 10 | 61.9 | 3.5 |
| Ex. 35 | FCV | 107 | 10 | 62.8 | ≥5.1 # |

| | | | | | |
|---|---|---|---|---|---|
| # below DL (limit of detection) | | | | | |

The results demonstrate significant inactivation of the full range of the selected model viruses. The proccss/process conditions according to the process as described herein provide for a significant inactivation of as high as 3 log₁₀ (LRV of 3) of highly resistant viruses (PPV) and very efficient inactivation of medium to highly resistant viruses (FCV and Reo3). Enveloped viruses (PsRV) and also the non-enveloped viruses Reo3 and FCV were all inactivated below the limit of detection (DL).

### Melt granulation

Samples for determining residual viral load after application of a melt granulation process were prepared as described above. Analytical testing of the samples was performed as described above for melt extrusion samples but using the samples drawn from the reaction vessel for the melt granulation process instead of the extrudates.

For each virus inactivation experiment Examples (36-49). 6 g API (porcine pancreatin powder, therapeutic grade) were homogenized with 1 g of lyophilized virus preparation of the corresponding virus type. The spiked API was mixed with a receiving melt made from 2 g Gelucire^{®} 44/14 and 2 g PEG 4000, as described above.

Results from viral analytics of the melt-granulated samples are shown in tables 2a-2c.

### 2, General method for determination of recovered enzyme activity

The enzyme activities in samples after processing (melt granulation or melt extrusion, as applicable) have been determined in accordance with the following procedures. Results are provided above (see e.g. tables 6 and 7 and section "melt granulation")

Samples for determining enzymatic activities (lipolytic. proteolytic or amylolytic, as applicable) were usually taken directly from the manufacturing process and. after cooling to room temperature, were used without further treatment, i.e. as extrudates in case of extrusion, or from the reaction vessel in case of melt granulation (see above). In some cases, extrudates were further processed to test samples by rounding before determination of enzymatic activity. As could be shown, rounding had no or no relevant influence on enzymatic activities.

All tests for enzymatic activities were performed in accordance with relevant methods of the Ph.Eur.. "pancreas powder". All enzymatic activities measured are given in "U/g". Enzymatic activities of the pancreatin-containing test samples were determined as follows:
Enzymatic activities of the pancreatin powder (therapeutical grade) which was used for producing the test samples were determined according to Ph. Eur. relative to the relevant reference standards (see below for details) and taken as initial enzymatic (lipolytic, proteolytic, amylolytic, as applicable) activities. Then, test samples were produced as disclosed herein (usually as granules or extrudates) using pancreatin from the same batches as used for determining initial enzyme activities. Resulting enzyme activities of test samples were then calculated based on the relative (% weight by weight) contents of pancreatin in the test samples, test samples further containing additional components like surfactant(s), co-surfactant(s), lipophilic phasc(s), polymeric additive(s) and/or further pharmaceutically acceptable agents (auxiliaries), as disclosed herein.

By way of example for calculating lipase (lipolytic) activities of pancreatin and test sample, initial lipase activity in a pancreatin powder (therapeutical grade) is determined relative to the lipase reference standard (see Ph. Eur.) and found to be e.g. 70000 U/g. Pancreatin powder from that same batch is then used to produce test samples, e.g. in the form of extrudates. Composition of extrudates is e.g. 60 % w/w pancreatin and 40 % w/w other components (as disclosed herein). This method is further explained in the exemplary calculation method below:
- Initial lipase activity in pancreatin as determined: 70000 U/g;
- Lipase activity expected in test sample after processing: 42000 U/g (60 % of 70000 U/g),
- Lipase activity found in test sample after processing: 40000 U/g.
- Remaining lipase activity in test sample after processing: 95 % (100*40000U/g / 42000 U/g).

The methods applied for determining enzymatic activities are standard in the art and show a generally accepted variance of about 5 % for all enzymatic activities tested, thus potentially resulting in measured values of above 100 % enzyme activity. This variance may be due to double impact of method variabilities of the Ph. Eur assay (at enzyme, e.g. lipase, activity determination in the drug substance and at enzyme, e.g. lipase, activity determination in the drug product).

Determination of lipase activity: an aliquot of a pancreatin-containing test sample as set out above was taken (about 2-5 g) and used for the subsequent analysis. The required amount of the test sample used for the lipase activity determination (depending on the activity expected) was comminuted (e.g. crushed or ground, depending on state/consistency of test sample) and directly extracted with buffer solution from the test sample as described in Ph. Eur. With this standard analysis method, the hydrolytic activity of the lipase in the sample to be investigated is determined with olive oil emulsion as substrate. The free fatty acids cleaved off from the triglycerides of the olive oil are titrated with sodium hydroxide solution at a constant pH of 9.0. The lipase activity of the sample is determined by comparing the rate at which a suspension of the pancreatin-containing sample hydrolyses a substrate of olive oil emulsion with the rate at which a suspension of a standard pancreas reference powder (reference standard of Ph.Eur. as described in the monograph, "pancreas powder (lipase) BRP") hydrolyses the same substrate under the same conditions.

Determination of amylase activity: an aliquot of a pancreatin-containing test sample as set out above was taken (about 2-5 g) and used for the subsequent analysis as described above for the lipase activity. The amylolytic activity was in each case determined by comparing the rate at which a suspension of amylase containing suspension hydrolyses a substrate of starch solution with the rate at which a suspension of reference standard hydrolyses the same substrate under the same conditions. The determination of amylase is based on the hydrolysis of starch. Starch is hydrolysed by amylase at pH 6.8 and at constant temperature (25.0 +/- 0.1 °C) in the presence of sodium chloride. The reducing groups resulting from the hydrolysis react with iodine in alkaline solution and the excess is titrated with thiosulphate.

Determination of protease activity: an aliquot of a pancreatin-containing test sample as set out above was taken (about 2-5 g) and used for die subsequent analysis as described above for the lipase activity. The proteolytic activity was determined by comparing the quantity of peptides (non-precipitable by a 50g/L. solution of trichloroacetic acid as defined in the monograph of pancreas powder in Ph. Eur.) released per minute from a substrate of casein solution with the quantity of such peptides released by pancreas powder ("protease BRP") reference standard from the same substrate under the same conditions. Casein is hydrolysed by protease at pH 7.5 and at a temperature of 35 °C +/- 0.5 °C for a defined time (15 minutes).

## Claims

1. A process for the preparation of a solid or semi-solid composition comprising processing a mixture, which mixture comprises:
(a) 40-75 % by weight of pancreatin and/or a pancreatin-containing mixture of digestive enzymes derived from a mammal;
(b) 10-50 % by weight of a surfactant-component comprising
(i) 2-90 % by weight, relative to the surfactant-component, of at least one surfactant selected from (aa) non-ionic surfactants, comprising polyethylene glycol fatty acid mono- and/or di-esters with aliphatic C₆-C₂₂-carboxylic acids; polyethylene glycol glycerol fatty acid esters with aliphatic C₆-C₂₂-carboxylic acids; polyethylene glycol alkyl mono- and/or di-ethers with aliphatic C12-C18-alcohols, oligoethylene glycol ethers with aliphatic C₂-C₁₈-alcohols; and mixtures of any of the foregoing, and (bb) ionic surfactants, comprising lecithin, lysolecithin, phosphatidylcholine, phosphatidylethanolamine, phosphatidylglycerol, phosphatidylserine, lysophosphatidylcholine, lysophosphatidylethanol-amine, lysophosphatidylglycerol, lysophosphatidylinositol, lyso-phosphatidic acid, lysophosphatidylserine; and mixtures of any of the foregoing, and mixtures of any of the foregoing surfactants from (aa) and (bb),
(ii) 5-60 % by weight, relative to the surfactant-component, of at least one co-surfactant selected from mono-acylglycerides with aliphatic C₆-C₂₂- carboxylic acids, mono-ethers of glycerol with aliphatic C₁₂-C22-alcohols, oligoethylene glycol monoesters with aliphatic C6- C22-carboxylic acids, oligoethylene glycol diesters with aliphatic C₆- C₂₂-carboxylic acids and mixtures of any of the foregoing, and
(iii) 0-70 % by weight, relative to the surfactant-component, of a lipophilic phase selected from di- and triacylglycerides with aliphatic C₆-C₂₂-carboxylic acids or mixtures of any of the foregoing, wherein the percentages (i), (ii) and (iii) add to 100 % by weight for the surfactant-component in each case;
(c) one or more antioxidant(s) present in a concentration of 50 to 200 ppm relative to the total weight of the solid or semi-solid composition, and
(d) 0-35% by weight of a polymeric additive selected from hydrophilic polymers with melting points or glass transition temperatures of 50-70 °C;
wherein the % by weight of components (a), (b), (c) and (d) add to 100 % by weight for said mixture;
wherein the weight ratio of the polymeric additive of (d) and the surfactant-component of (b) is 0.4 (2:5) to 1.5 (3:2); and the mixture is subjected to a method selected from melt granulation, melt pelletization and melt extrusion at a temperature of 90-130 °C and for a time period between 30 seconds and 45 minutes.

2. The process according to claim 1, wherein the mixture for preparing a solid composition comprises:
(a) 40-70 % by weight of the pancreatin and/or pancreatin containing mixture of digestive enzymes derived from a mammal;
(b) 15-45 % by weight of the surfactant-component,
(c) 50 to 200 ppm of the one or more antioxidant(s) relative to the total weight of the solid or semi-solid composition, and
(d) 10-30 % by weight of the polymeric additive;
and wherein the % by weight of components (a), (b), (c) and (d) add to 100 % by weight for said mixture.

3. The process according to claim 1 or claim 2, wherein the mixture for preparing a solid composition is homogenized before processing and/or during processing.

4. A process according to any one of claims 1 to 3, wherein component (a) is porcine pancreatin in an amount of 64 % +/- 6 % by weight of the mixture and components (b), (c), and (d) together are present in an amount of 36 % +/- 6 % by weight of the mixture.

5. A process according to any one of claims 1 to 4, wherein:
(1) the surfactant-component (b) is semisynthetic lauroyl macrogol-32 glycerides on the basis of hydrogenated palm kernel oil having a melting point of about 42.5-47.5 °C
(2) and the polymeric additive (d) is polyethylene glycol 4000,
(3) (b) and (d) make up 30-42% by weight of the mixture and are present in a ratio of 1:1 by weight, and
(4) the composition further comprises 150 ppm butylated hydroxyanisole (BHA), relative to the combined total weights of components (b) and (d).

6. The process according to any one of claims 1 to 5, wherein the mixture is melt extruded using single-screw extruders, twin-screw-extruders, triple-screw extruders, or planetary extruders.

7. The process according to claim 6, wherein the mixture for the preparation of a solid composition is processed at a product temperature between 95 °C and 125 °C for a time period between 50 seconds and 10 minutes.

8. The process according to any one of claims 1 to 5, wherein the mixture is melt granulated at a temperature between 90 °C and 125 °C for a time period between 180 seconds and 30 minutes.

9. A solid or semi-solid composition comprising:
(a) 40-75% by weight of the composition of pancreatin and/or a pancreatin containing mixture of digestive enzymes derived from a mammal;
(b) 10-50 % by weight of the composition of a surfactant-component of
(i) 2-90 % by weight, relative to the surfactant-component, of at least one surfactant selected from (aa) non-ionic surfactants, comprising polyethylene glycol fatty acid mono- and/or di-esters with aliphatic C₆-C₂₂-carboxylic acids; polyethylene glycol glycerol fatty acid esters with aliphatic C₆-C₂₂-carboxlic acids; polyethylene glycol alkyl mono- and/or di-ethers with aliphatic C12-C18-alcohols, oligoethylene glycol ethers with aliphatic C₂-C₁₈-alcohols; and mixtures of any of the foregoing, and (bb) ionic surfactants, comprising lecithin, lysolecithin, phosphatidylcholine, phosphatidylethanolamine, phosphatidylglycerol, phosphatidylserine, lysophosphatidylcholine, lysophosphatidylethanol- amine, lysophosphatidylglycerol, lysophosphatidylinositol, lyso-phosphatidic acid, lysophosphatidylserine; and mixtures of any of the foregoing, and mixtures of any of the foregoing surfactants from (aa) and (bb);
(ii) 5-60 % by weight, relative to the surfactant-component, of at least one co-surfactant selected from mono-acylglycerides with aliphatic C6-C₂₂- carboxylic acids, mono-ethers of glycerol with aliphatic C₁₂-C22- alcohols, partial esters of propylenglycol with aliphatic C₆-C₂₂-carboxylic acids, partial esters of polyglycerol with aliphatic C₆-C₂₂-carboxylic acids, oligoethylene glycol monoesters with aliphatic C6-C₂₂-carboxylic acids, oligoethylene glycol diesters with aliphatic C₆-C₂₂-carboxylic acids, and mixtures of any of the foregoing, and
(iii) 0-70 % by weight, relative to the surfactant-component, of a lipophilic phase selected from di- and triacylglycerides with aliphatic C₆-C₂₂-carboxylic acids, and/or mixtures of any of the foregoing;
wherein the percentages (i), (ii) and (iii) add to 100 % by weight for the surfactant-component;
(c) one or more antioxidant(s), present in a concentration of 50 to 200 ppm relative to the total weight of the solid or semi-solid composition; and
(d) 5-50 % by weight of the composition of a polymeric additive selected from hydrophilic polymers with melting points or glass transition temperatures of 50-160 °C;
wherein (i) the ratio of the polymeric additive (d) and the surfactant-component (b) is 0.4 (2:5) to 1.5 (3:2), (ii) all weight percentages of components (a), (b), (c) and (d) in the composition add to 100% by weight, and (iii) the solid or semi-solid composition being prepared by subjecting the mixture of components (a) to (d) to a method selected from melt granulation, melt pelletization and melt extrusion at a temperature of 90 -130 °C and for a time period between 30 seconds and 45 minutes.

10. A composition according to claim 9, wherein the pancreatin and/or pancreatin containing mixture of digestive enzymes derived from a mammal is porcine pancreatin.

11. A composition according to claim 9 or claim 10, wherein the polymeric additive (d) is selected from hydrophilic polymers with melting points or glass transition temperatures of 50-65°C.

12. A composition according to any one of claims 9-11, wherein the polymeric additive is selected from PEG 20000, PEG 4000 and Poloxamer 188.

13. A composition according to any one of claims 9-12, wherein the pancreatin and/or pancreatin containing mixture of digestive enzymes derived from a mammal is present in an amount of 40-70 % by weight of the composition.

14. A composition according to any one of claims 9-13, wherein the surfactant-component (b) is present in an amount of 15-40 % by weight of the composition.

15. A composition according to any one of claims 9-14, wherein the polymeric additive (d) is present in an amount of 5-35 % by weight of the composition.

16. A composition according to any one of claims 9-15, wherein the surfactant-component (b) is selected from semisynthetic lauroyl macrogol-32 glycerides on the basis of hydrogenated palm kernel oil having a melting point of about 42.5-47.5 °C and comprising mono- and diesters of polyethylene glycol 1500 at about 72 wt.-% , mono-, di- and triglycerides of fatty acids 20 wt.-% and free polyethylene glycol 1500 about 8 wt.-%, free glycerol < wt.-3%, wherein the apportionment of the fatty acids is: C8< 15 wt.-%, C10< 12 wt.-%, C12<30-50 wt.-%, C14 5-25 wt.-%, C16 4-25 wt.-% and C18 5-35 wt.-%.

17. A pharmaceutical composition comprising the composition of any one of claims 9-16 and pharmaceutically acceptable excipients.

18. The pharmaceutical composition according to claim 17 for the use in the prophylaxis or treatment of digestive disorders, pancreatic exocrine insufficiency, pancreatitis, cystic fibrosis, diabetes type I and/or diabetes type II.

## Patentansprüche

1. Ein Verfahren zur Herstellung einer festen oder halbfesten Zusammensetzung, umfassend Verarbeiten eines Gemischs, wobei das Gemisch umfasst:
(a) 40-75 Gew.-% Pankreatin und/oder eines Pankreatin enthaltenden Gemischs von Verdauungsenzymen, die von einem Säuger stammen;
(b) 10-50 Gew.-% einer Tensidkomponente, umfassend
(i) 2-90 Gew.-%, bezogen auf die Tensidkomponente, mindestens eines Tensids, ausgewählt aus (aa) nichtionischen Tensiden, umfassend Polyethylenglycol-Fettsäuremono- und/oder -diester mit aliphatischen C₆-C₂₂-Carbonsäuren; Polyethylenglycol-Glycerinfettsäureester mit aliphatischen C₆-C₂₂-Carbonsäuren; Polyethylenglycol-Alkylmono- und/oder -diether mit aliphatischen C₁₂-C₁₈-Alkoholen, Oligoethylenglycolether mit aliphatischen C₂-C₁₈-Alkoholen; und Gemische von beliebigen der vorgenannten, und (bb) ionischen Tensiden, umfassend Lecithin, Lysolecithin, Phosphatidylcholin, Phosphatidylethanolamin, Phosphatidylglycerin, Phosphatidylserin, Lysophosphatidylcholin, Lysophosphatidylethanolamin, Lysophosphatidylglycerin, Lysophosphatidylinositol, Lysophosphatidsäure, Lysophosphatidylserin und Gemische von beliebigen der vorgenannten, und Gemische von beliebigen der vorgenannten Tenside aus (aa) und (bb),
(ii) 5-60 Gew.-%, bezogen auf die Tensidkomponente, mindestens eines Co-Tensids, ausgewählt aus Monoacylglyceriden mit aliphatischen C₆-C₂₂-Carbonsäuren, Monoethern von Glycerin mit aliphatischen C₁₂-C₂₂-Alkoholen, Oligoethylenglycolmonoestern mit aliphatischen C₆-C₂₂-Carbonsäuren, Oligoethylenglycoldiestern mit aliphatischen C₆-C₂₂-Carbonsäuren und Gemischen von beliebigen der vorgenannten, und
(iii) 0-70 Gew.-%, bezogen auf die Tensidkomponente, einer lipophilen Phase, ausgewählt aus Di- und Triacylglyceriden mit aliphatischen C₆-C₂₂-Carbonsäuren oder Gemischen von beliebigen der vorgenannten, wobei die Prozentsätze (i), (ii) und (iii) in jedem Fall zusammen 100 Gew.-% für die Tensidkomponente ergeben;
(c) ein oder mehrere Antioxidationsmittel, die in einer Konzentration von 50 bis 200 ppm, bezogen auf das Gesamtgewicht der festen oder halbfesten Zusammensetzung, vorliegen, und
(d) 0-35 Gew.-% eines polymeren Additivs, das aus hydrophilen Polymeren mit Schmelzpunkten oder Glasübergangstemperaturen von 50-70°C ausgewählt ist;
wobei die Gewichts-% der Komponenten (a), (b), (c) und (d) zusammen 100 Gew.-% für das Gemisch ergeben;
wobei das Gewichtsverhältnis des polymeren Additivs (d) und der Tensidkomponente (b) 0,4 (2:5) bis 1,5 (3:2) beträgt; und das Gemisch einem Verfahren unterzogen wird, das aus Schmelzgranulation, Schmelzpelletierung und Schmelzextrusion bei einer Temperatur von 90-130°C und für einen Zeitraum zwischen 30 Sekunden und 45 Minuten ausgewählt ist.

2. Das Verfahren nach Anspruch 1, wobei das Gemisch zur Herstellung einer festen Zusammensetzung umfasst:
(a) 40-70 Gew.-% des Pankreatins und/oder eines Pankreatin enthaltenden Gemischs von Verdauungsenzymen, die von einem Säuger stammen;
(b) 15-45 Gew.-% der Tensidkomponente,
(c) 50 bis 200 ppm des einen oder der mehreren Antioxidationsmittel, bezogen auf das Gesamtgewicht der festen oder halbfesten Zusammensetzung, und
(d) 10 bis 30 Gew.-% des polymeren Additivs;
und wobei die Gewichts-% der Komponenten (a), (b), (c) und (d) zusammen 100 Gew.-% für das Gemisch ergeben.

3. Das Verfahren nach Anspruch 1 oder Anspruch 2, wobei das Gemisch zur Herstellung einer festen Zusammensetzung vor der Verarbeitung und/oder während der Verarbeitung homogenisiert wird.

4. Ein Verfahren nach einem der Ansprüche 1 bis 3, wobei Komponente (a) Schweinepankreatin in einer Menge von 64 Gew.-% +/- 6 Gew.-% des Gemischs ist und die Komponenten (b), (c) und (d) zusammen in einer Menge von 36 Gew.-% +/- 6 Gew.-% des Gemischs vorliegen.

5. Ein Verfahren nach einem der Ansprüche 1 bis 4, wobei:
(1) die Tensidkomponente (b) halbsynthetische Lauroyl-Macrogol-32-Glyceride auf der Basis von hydriertem Palmkernöl mit einem Schmelzpunkt von etwa 42,5 bis 47,5°C ist
(2) und das polymere Additiv (d) Polyethylenglycol 4000 ist,
(3) (b) und (d) 30-42 Gew.-% des Gemischs ausmachen und in einem Gewichtsverhältnis von 1:1 vorliegen und
(4) die Zusammensetzung ferner 150 ppm butyliertes Hydroxyanisol (BHA), bezogen auf die kombinierten Gesamtgewichte der Komponenten (b) und (d), umfasst.

6. Das Verfahren nach einem der Ansprüche 1 bis 5, wobei das Gemisch unter Verwendung von Einschneckenextrudern, Doppelschneckenextrudern, Dreifachschneckenextrudern oder Planetenextrudern schmelzextrudiert wird.

7. Das Verfahren nach Anspruch 6, wobei das Gemisch zur Herstellung einer festen Zusammensetzung bei einer Produkttemperatur zwischen 95°C und 125°C für einen Zeitraum zwischen 50 Sekunden und 10 Minuten verarbeitet wird.

8. Das Verfahren nach einem der Ansprüche 1 bis 5, wobei das Gemisch bei einer Temperatur zwischen 90°C und 125°C für einen Zeitraum zwischen 180 Sekunden und 30 Minuten schmelzgranuliert wird.

9. Eine feste oder halbfeste Zusammensetzung, umfassend:
(a) 40-75 Gew.-% der Zusammensetzung Pankreatin und/oder eine Pankreatin enthaltende Mischung von Verdauungsenzymen, die von einem Säuger stammen;
(b) 10-50 Gew.-% der Zusammensetzung einer Tensidkomponente aus
(i) 2-90 Gew.-%, bezogen auf die Tensidkomponente, mindestens eines Tensids, ausgewählt aus (aa) nichtionischen Tensiden, umfassend Polyethylenglycol-Fettsäuremono- und/oder -diester mit aliphatischen C₆-C₂₂-Carbonsäuren; Polyethylenglycol-Glycerinfettsäureester mit aliphatischen C₆-C₂₂-Carbonsäuren; Polyethylenglycolalkylmono- und/oder -diether mit aliphatischen C₁₂-C₁₈-Alkoholen, Oligoethylenglykolether mit aliphatischen C₂-C₁₈-Alkoholen; und Gemische von beliebigen der vorgenannten, und (bb) ionischen Tensiden, umfassend Lecithin, Lysolecithin, Phosphatidylcholin, Phosphatidylethanolamin, Phosphatidylglycerin, Phosphatidylserin, Lysophosphatidylcholin, Lysophosphatidylethanolamin, Lysophosphatidylglycerin, Lysophosphatidylinositol, Lysophosphatidsäure, Lysophosphatidylserin; und Gemische von beliebigen der vorgenannten, und Gemische von beliebigen der vorgenannten Tenside aus (aa) und (bb);
(ii) 5-60 Gew.-%, bezogen auf die Tensidkomponente, mindestens eines Co-Tensids, ausgewählt aus Monoacylglyceriden mit aliphatischen C₆-C₂₂-Carbonsäuren, Monoethern von Glycerin mit aliphatischen C₁₂-C₂₂-Alkoholen, Teilestern von Propylenglycol mit aliphatischen C₆-C₂₂-Carbonsäuren, Teilestern von Polyglycerin mit aliphatischen C₆-C₂₂-Carbonsäuren, Oligoethylenglycolmonoestern mit aliphatischen C₆-C₂₂-Carbonsäuren, Oligoethylenglycoldiestern mit aliphatischen C₆-C₂₂-Carbonsäuren und Gemische von beliebigen der vorgenannten, und
(iii) 0-70 Gew.-%, bezogen auf die Tensidkomponente, einer lipophilen Phase, ausgewählt aus Di- und Triacylglyceriden mit aliphatischen C₆-C₂₂-Carbonsäuren und/oder Gemischen von beliebigen der vorgenannten;
wobei die Prozentsätze (i), (ii) und (iii) zusammen 100 Gew.-% für die Tensidkomponente ergeben;
(c) ein oder mehrere Antioxidationsmittel, die in einer Konzentration von 50 bis 200 ppm, bezogen auf das Gesamtgewicht der festen oder halbfesten Zusammensetzung, vorliegen; und
(d) 5-50 Gew.-% der Zusammensetzung eines polymeren Additivs, das aus hydrophilen Polymeren mit Schmelzpunkten oder Glasübergangstemperaturen von 50-160°C ausgewählt ist;
wobei (i) das Verhältnis des polymeren Additivs (d) und der Tensidkomponente (b) 0,4 (2:5) bis 1,5 (3:2) beträgt, (ii) alle Gewichtsprozente der Komponenten (a), (b), (c) und (d) in der Zusammensetzung zusammen 100 Gew.-% ergeben und (iii) die feste oder halbfeste Zusammensetzung hergestellt wird, indem das Gemisch der Komponenten (a) bis (d) einem Verfahren unterzogen wird, das aus Schmelzgranulation, Schmelzpelletierung und Schmelzextrusion bei einer Temperatur von 90-130°C und für einen Zeitraum zwischen 30 Sekunden und 45 Minuten ausgewählt ist.

10. Eine Zusammensetzung nach Anspruch 9, wobei das Pankreatin und/oder das Pankreatin enthaltende Gemisch von Verdauungsenzymen, die von einem Säuger stammen, Schweinepankreatin ist.

11. Eine Zusammensetzung nach Anspruch 9 oder Anspruch 10, wobei das polymere Additiv (d) aus hydrophilen Polymeren mit Schmelzpunkten oder Glasübergangstemperaturen von 50-65°C ausgewählt ist.

12. Eine Zusammensetzung nach einem der Ansprüche 9-11, wobei das polymere Additiv aus PEG 20000, PEG 4000 und Poloxamer 188 ausgewählt ist.

13. Eine Zusammensetzung nach einem der Ansprüche 9-12, wobei das Pankreatin und/oder das Pankreatin enthaltende Gemisch von Verdauungsenzymen, die von einem Säuger stammen, in einer Menge von 40-70 Gew.-% der Zusammensetzung vorliegt.

14. Eine Zusammensetzung nach einem der Ansprüche 9-13, wobei die Tensidkomponente (b) in einer Menge von 15-40 Gew.-% der Zusammensetzung vorliegt.

15. Eine Zusammensetzung nach einem der Ansprüche 9-14, wobei das polymere Additiv (d) in einer Menge von 5-35 Gew.-% der Zusammensetzung vorliegt.

16. Eine Zusammensetzung nach einem der Ansprüche 9-15, wobei die Tensidkomponente (b) aus halbsynthetischen Lauroyl-Macrogol-32-Glyceriden auf der Basis von hydriertem Palmkernöl mit einem Schmelzpunkt von etwa 42,5-47,5°C ausgewählt ist und Mono- und Diester von Polyethylenglycol 1500 in etwa 72 Gew.-%, Mono-, Di- und Triglyceride von Fettsäuren in 20 Gew.-% und freies Polyethylenglycol 1500 in etwa 8 Gew.-%, freies Glycerin in < Gew.-3% umfasst, wobei die Aufteilung der Fettsäuren: C8 < 15 Gew.-%, C10 < 12 Gew.-%, C12 < 30-50 Gew.-%, C14 5-25 Gew.-%, C16 4-25 Gew.-% und C18 5-35 Gew.-% ist.

17. Ein Arzneimittel, umfassend die Zusammensetzung nach einem der Ansprüche 9-16 und pharmazeutisch verträgliche Exzipienten.

18. Das Arzneimittel nach Anspruch 17 zur Verwendung bei der Prophylaxe oder Behandlung von Verdauungsstörungen, exokriner Pankreasinsuffizienz, Pankreatitis, zystischer Fibrose, Diabetes Typ I und/oder Diabetes Typ II.

## Revendications

1. Procédé pour la préparation d'une composition solide ou semi-solide, comprenant le traitement d'un mélange, lequel mélange comprend :
(a) 40 à 75 % en poids de pancréatine et/ou d'un mélange, contenant de la pancréatine, d'enzymes digestives dérivées d'un mammifère ;
(b) 10 à 50 % en poids d'un composant tensioactif comprenant
(i) 2 à 90 % en poids, par rapport au composant tensioactif, d'au moins un tensioactif choisi parmi (aa) les tensioactifs non-ioniques, comprenant les mono- et/ou di-esters d'acide gras et de polyéthylèneglycol avec des acides carboxyliques aliphatiques en C₆ à C₂₂ ; les esters d'acide gras et de polyéthylèneglycol et de glycérol avec des acides carboxyliques aliphatiques en C₆ à C₂₂ ; les mono- et/ou di-éthers alkyliques de polyéthylèneglycol avec des alcools aliphatiques en C₁₂ à C₁₈, les éthers d'oligoéthylèneglycol avec des alcools aliphatiques en C₂ à C₁₈ ; et les mélanges de n'importe lesquels des précédents, et (bb) les tensioactifs ioniques, comprenant la lécithine, la lysolécithine, la phosphatidylcholine, la phosphatidyléthanolamine, le phosphatidylglycérol, la phosphatidylsérine, la lysophosphatidylcholine, la lysophosphatidyléthanolamine, le lysophosphatidylgycérol, le lysophosphatidylinositol, l'acide lysophosphatidique, la lysophosphatidylsérine ; et les mélanges de n'importe lesquels des précédents, et les mélanges de n'importe lesquels des tensioactifs précédents de (aa) et (bb),
(ii) 5 à 60 % en poids, par rapport au composant tensioactif, d'au moins un co-tensioactif choisi parmi les monoacylglycérides avec des acides carboxyliques aliphatiques en C₆ à C₂₂, les monoéthers de glycérol avec des alcools aliphatiques en C₁₂ à C₂₂, les monoesters d'oligoéthylèneglycol avec des acides carboxyliques aliphatiques en C₆ à C₂₂, les diesters d'oligoéthylèneglycol avec des acides carboxyliques aliphatiques en C₆ à C₂₂, et les mélanges de n'importe lesquels des précédents, et
(iii) 0 à 70 % en poids, par rapport au composant tensioactif, d'une phase lipophile choisie parmi les di- et tri-acylglycérides avec des acides carboxyliques aliphatiques en C₆ à C₂₂ et les mélanges de n'importe lesquels des précédents, dans lesquels les pourcentages de (i), (ii) et (iii) s'additionnent à 100 % en poids pour le composant tensioactif dans chaque cas ;
(c) un ou plusieurs antioxydants présents à une concentration de 50 à 200 ppm par rapport au poids total de la composition solide ou semi-solide, et
(d) 0 à 35 % en poids d'un additif polymère choisi parmi les polymères hydrophiles ayant des points de fusion ou des températures de transition vitreuse de 50 à 70 °C ;
dans lequel les pourcentages en poids des composants (a), (b), (c) et (d) s'additionnent à 100 % en poids pour ledit mélange ;
dans lequel le rapport en poids de l'additif polymère de (d) au composant tensioactif de (b) est de 0,4 (2/5) à 1,5 (3/2), et le mélange est soumis à une méthode choisie parmi une granulation à l'état fondu, un pastillage à l'état fondu et une extrusion à l'état fondu à une température de 90 à 130 °C et pendant une période de temps comprise entre 30 secondes et 45 minutes.

2. Procédé selon la revendication 1, dans lequel le mélange pour préparer une composition solide comprend :
(a) 40 à 70 % en poids de la pancréatine et/ou du mélange, contenant de la pancréatine, d'enzymes digestives dérivées d'un mammifère ;
(b) 15 à 45 % en poids du composant tensioactif,
(c) 50 à 200 ppm du ou des antioxydants par rapport au poids total de la composition solide ou semi-solide, et
(d) 10 à 30 % en poids de l'additif polymère ;
et dans lequel les pourcentages en poids des composants (a), (b), (c) et (d) s'additionnent à 100 % en poids pour ledit mélange.

3. Procédé selon la revendication 1 ou la revendication 2, dans lequel le mélange pour préparer une composition solide est homogénéisée avant le traitement et/ou pendant le traitement.

4. Procédé selon l'une quelconque des revendications 1 à 3, dans lequel le composant (a) est la pancréatine de porc en une quantité de 64 % ± 6 % en poids du mélange et les composants (b), (c) et (d) ensemble sont présents en une quantité de 36 % ± 6 % en poids du mélange.

5. Procédé selon l'une quelconque des revendications 1 à 4, dans lequel :
(1) le composant tensioactif (b) consiste en glycérides de lauroyl-macrogol-32 semi-synthétiques à base d'huile de palmiste hydrogénée ayant un point de fusion d'environ 42,5 à 47,5 °C,
(2) et l'additif polymère (d) est le polyéthylèneglycol 4000,
(3) (b) et (d) constituent 30 à 42 % en poids du mélange et sont présents en un rapport de 1/1 en poids, et
(4) la composition comprend en outre 150 ppm d'hydroxyanisole butylé (BHA), par rapport aux poids totaux combinés des composants (b) et (d).

6. Procédé selon l'une quelconque des revendications 1 à 5, dans lequel le mélange est extrudé à l'état fondu au moyen d'extrudeuses simple vis, d'extrudeuses double vis, d'extrudeuses triple vis, ou d'extrudeuses planétaires.

7. Procédé selon la revendication 6, dans lequel le mélange pour la préparation d'une composition solide est traitée à une température de produit comprise entre 95 °C et 125 °C pendant une période de temps comprise entre 50 secondes et 10 minutes.

8. Procédé selon l'une quelconque des revendications 1 à 5, dans lequel le mélange est granulé à l'état fondu à une température comprise entre 90 °C et 125 °C pendant une période de temps comprise entre 180 secondes et 30 minutes.

9. Composition solide ou semi-solide comprenant :
(a) 40 à 75 %, en poids de la composition, de pancréatine et/ou d'un mélange, contenant de la pancréatine, d'enzymes digestives dérivées d'un mammifère ;
(b) 10 à 50 %, en poids de la composition, d'un composant tensioactif consistant en
(i) 2 à 90 % en poids, par rapport au composant tensioactif, d'au moins un tensioactif choisi parmi (aa) les tensioactifs non-ioniques, comprenant les mono- et/ou di-esters d'acide gras et de polyéthylèneglycol avec des acides carboxyliques aliphatiques en C₆ à C₂₂ ; les esters d'acide gras et de polyéthylèneglycol et de glycérol avec des acides carboxyliques aliphatiques en C₆ à C₂₂ ; les mono- et/ou di-éthers alkyliques de polyéthylèneglycol avec des alcools aliphatiques en C₁₂ à C₁₈, les éthers d'oligoéthylèneglycol avec des alcools aliphatiques en C₂ à C₁₈ ; et les mélanges de n'importe lesquels des précédents, et (bb) les tensioactifs ioniques, comprenant la lécithine, la lysolécithine, la phosphatidylcholine, la phosphatidyléthanolamine, le phosphatidylglycérol, la phosphatidylsérine, la lysophosphatidylcholine, la lysophosphatidyléthanolamine, le lysophosphatidylgycérol, le lysophosphatidylinositol, l'acide lysophosphatidique, la lysophosphatidylsérine ; et les mélanges de n'importe lesquels des précédents, et les mélanges de n'importe lesquels des tensioactifs précédents de (aa) et (bb) ;
(ii) 5 à 60 % en poids, par rapport au composant tensioactif, d'au moins un co-tensioactif choisi parmi les monoacylglycérides avec des acides carboxyliques aliphatiques en C₆ à C₂₂, les monoéthers de glycérol avec des alcools aliphatiques en C₁₂ à C₂₂, les esters partiels de propylèneglycol avec des acides carboxyliques aliphatiques en C₆ à C₂₂, les esters partiels de polyglycérol avec des acides carboxyliques aliphatiques en C₆ à C₂₂, les monoesters d'oligoéthylèneglycol avec des acides carboxyliques aliphatiques en C₆ à C₂₂, les diesters d'oligoéthylèneglycol avec des acides carboxyliques aliphatiques en C₆ à C₂₂, et les mélanges de n'importe lesquels des précédents, et
(iii) 0 à 70 % en poids, par rapport au composant tensioactif, d'une phase lipophile choisie parmi les di- et tri-acylglycérides avec des acides carboxyliques aliphatiques en C₆ à C₂₂ et les mélanges de n'importe lesquels des précédents,
dans laquelle les pourcentages de (i), (ii) et (iii) s'additionnent à 100 % en poids pour le composant tensioactif ;
(c) un ou plusieurs antioxydants présents à une concentration de 50 à 200 ppm par rapport au poids total de la composition solide ou semi-solide ; et
(d) 5 à 50 %, en poids de la composition, d'un additif polymère choisi parmi les polymères hydrophiles ayant des points de fusion ou des températures de transition vitreuse de 50 à 160 °C ;
dans laquelle (i) le rapport en poids de l'additif polymère (d) au composant tensioactif (b) est de 0,4 (2/5) à 1,5 (3/2), (ii) tous les pourcentages en poids des composants (a), (b), (c) et (d) dans la composition s'additionnent à 100 % en poids, et (iii) la composition solide ou semi-solide est préparée par soumission du mélange des composants (a) à (d) à une méthode choisie parmi une granulation à l'état fondu, un pastillage à l'état fondu et une extrusion à l'état fondu à une température de 90 à 130 °C et pendant une période de temps comprise entre 30 secondes et 45 minutes.

10. Composition selon la revendication 9, dans laquelle la pancréatine et/ou le mélange, contenant de la pancréatine, d'enzymes digestives dérivées d'un mammifère est la pancréatine de porc.

11. Composition selon la revendication 9 ou la revendication 10, dans laquelle l'additif polymère (d) est choisi parmi les polymères hydrophiles ayant des points de fusion ou des températures de transition vitreuse de 50 à 65 °C.

12. Composition selon l'une quelconque des revendications 9 à 11, dans laquelle l'additif polymère est choisi parmi le PEG 20000, le PEG 4000 et le poloxamère 188.

13. Composition selon l'une quelconque des revendications 9 à 12, dans laquelle la pancréatine et/ou le mélange, contenant de la pancréatine, d'enzymes digestives dérivées d'un mammifère, sont présents en une quantité de 40 à 70 % en poids de la composition.

14. Composition selon l'une quelconque des revendications 9 à 13, dans laquelle le composant tensioactif (b) est présent en une quantité de 15 à 40 % en poids de la composition.

15. Composition selon l'une quelconque des revendications 9 à 14, dans laquelle l'additif polymère (d) est présent en une quantité de 5 à 35 % en poids de la composition.

16. Composition selon l'une quelconque des revendications 9 à 15, dans laquelle le composant tensioactif (b) est choisi parmi les glycérides de lauroyl-macrogol-32 semi-synthétiques à base d'huile de palmiste hydrogénée ayant un point de fusion d'environ 42,5 à 47,5 °C et comprenant des mono- et di-esters de polyéthylèneglycol 1500 à raison d'environ 72 % en poids, des mono-, di- et tri-glycérides d'acides gras à raison de 20 % en poids et du polyéthylèneglycol 1500 libre à raison d'environ 8 % en poids, du glycérol libre à raison de moins de 3 % en poids, dans laquelle la répartition des acides gras est la suivante : C8 < 15 % en poids, C10 < 12 % en poids, C12 < 30 à 50 % en poids, C14 5 à 25 % en poids, C16 4 à 25 % en poids et C18 5 à 35 % en poids.

17. Composition pharmaceutique comprenant la composition de l'une quelconque des revendications 9 à 16 et des excipients pharmaceutiquement acceptables.

18. Composition pharmaceutique selon la revendication 17, pour une utilisation dans la prophylaxie ou le traitement de troubles digestifs, d'une insuffisance exocrine pancréatique, d'une pancréatite, d'une fibrose kystique, d'un diabète de type 1 et/ou d'un diabète de type II.
